# EUROPEAN PATENT APPLICATION

(11) **EP 3 138 531 A1**
(43) Date of publication of application: **08.03.2017**
(21) Application number: 16171630.3
(22) Date of filing: 22.03.2006
(51) Int. Cl.: A61F 2/00, A61K 31/44, A61L 31/16

(54) **COMPOSITIONS AND METHODS OF ADMINISTERING RAPAMYCIN ANALOGS USING MEDICAL DEVICES FOR LONG-TERM EFFICACY**

(30) Priority: 23.03.2005 US 664488 P; 08.09.2005 US 715082 P; 14.10.2005 US 727080 P; 14.10.2005 US 726878 P; 14.10.2005 US 727196 P; 17.10.2005 US 732577 P
(62) Divisional of application: 06739478.3
(71) Applicant: Abbott Laboratories, Abbott Park, IL 60064-3502 (US)
(72) Inventor: TONER, John L., Libertyville, IL 60048 (US); BURKE, Sandra E., Monroe, NC 28110-9639 (US); CROMACK, KeithR, GURNEE, IL 60031 (US)
(74) Representative: ZBM Patents - Zea, Barlocci & Markvardsen

(57) **Abstract**

Systems and compositions comprising zotarolimus that are safer, more effective and produce less inflammation than rapamycin and paclitaxel systems are disclosed. Medical devices comprising supporting structures capable of containing or supporting a pharmaceutically acceptable carrier or excipient, which carrier or excipient can contain one or more therapeutic agents or substances, with the carrier including a coating on the surface thereof, and the coating having the therapeutic compounds, including, for example, drugs. Supporting structures for the medical devices that are suitable for use in this invention include coronary stents, peripheral stents, catheters, arterio-venous grafts, bypass grafts, and drug delivery balloons used in the vasculature. These compositions and systems can be used in combination with other drugs, including anti-proliterative agents, anti-platelet agents, anti-inflammatory agents, anti-thrombotic agents, cytotoxic drugs, agents that inhibit cytokine or chemokine binding, cell de-differentiation inhibitors, anti-lipaedemic agents, matrix metalloproteinase inhibitors, cytostatic drugs, or combinations of these and other drugs.

## Description

### Technical Field

The present invention relates to novel drug delivery systems having the characteristics of improved drug delivery to tissues, safety, and diminished inflammatory responses.

### Background of the Invention

### Stents

As blood travels through a vessel and reaches a constricted area, its progress is impeded, to the detriment of downstream tissues which depend on unimpeded flow for the delivery of nutrients, oxygen, and the pick-up of cell wastes. Enter the stent which purpose is to keep a body lumen, including a blood vessel, open. Stents are tubes made, for example, of expandable wire mesh or other perforated material. Stents are widely used in several other body lumens, not just blood vessels, including bile ducts, esophagus, trachea or large bronchi, ureters, and urethra. These life-saving devices bear the name of the English dentist, Charles Stent (1845-1901).

Stents were exploited in the late 1980's to maintain vessel patency after angioplasty. Stenting is involved in 90% of angioplasty performed today. Before the introduction of stents, the rate of restenosis ranged from 30% to 50% of the patients who were treated with balloon angioplasty. The recurrence rate after dilatation ot in-stent restenosis can be as high as 70% in selected patient subsets, while the angiographic restenosis rate in *de novo* stent placement is about 20%. Placement of the stent reduced the restenosis rate to 15% to 20%. This percentage likely represents the best results obtainable with purely mechanical stenting.

In surgery or other related invasive procedures, the insertion of a medical device having an interventional component including stents in blood vessels, urinary tracts or other difficult-to-access places for the purpose of preventing restenosis, providing vessel or lumen wall support or reinforcement and for other therapeutic or restorative functions has become a common form of long-term treatment. Typically, such intervention components are applied to a location of interest using a vascular catheter, or similar transluminal device, to carry the stent to the location of interest where it is thereafter released to expand or be expanded in situ. These devices are generally designed as permanent implants that may become incorporated in the vascular or other tissue that they contact at implantation.

Implanted interventional components including stents have also been used to carry medicinal agents, including thrombolytic agents. A thermal, memoried expanding plastic stent device that can be formulated to carry a medicinal agent by using the material of the stent itself as an inert polymeric drug carrier has been disclosed (Froix, 1992). Drug elution rates from a drug-loaded coating having a hydrophilic (or lipophobic) drug are usually very fast initially when the coated device contacts body fluid or blood. Thus, an ongoing problem for drug delivery stents is achieving therapeutic drug concentrations at a target site within the body with minimal losses and systemic side effects. One technique to reduce the so-called "burst effect" is to add a membrane that includes porosigen over the coating layer having the biologically active material (Eury et al., 1997; Helmus et al., 1995). Polymers are also used on stents as drug release coatings (Yang et al., 2002). Ding and Helmus describe elastomer coated implants in which the elastomer overcoat to control release of biologically active agent from an undercoat applied to a stent (Ding and Helmus, 2001). Tuch discloses a porous polymer on a stent to control the administration of a drug (Tuch, 1997). Kopia *et al.* describe a stent coated with a polymer loaded with a combination of drugs, including rapamycin and dexamethasone (Kopia et al., 2001). Pinchuk discloses a stent of a polymeric material that may be employed with a coating associated with the delivery of drugs (Pinchuk, 1992). Devices of the class using bio-degradable or bio-sorbable polymers have also been disclosed (MacGregor, 1991; Tang et al., 1990). Sahatjian discloses a coating applied to a stent consisting of a hydrogel polymer and a preselected drug; possible drugs include cell growth inhibitors and heparin (Sahatjian, 1994). A further method of making a coated intravascular stent carrying a therapeutic material in which a polymer coating is dissolved in a solvent and the therapeutic material dispersed in the solvent and the solvent thereafter evaporated (Berg et al., 1995).

Polymer/ drug/membrane systems, including those releasing heparin (Helmus, 1990) require two distinct layers to function. Ding and Helmus describe a process for coating a stent prosthesis using a biostable hydrophobic elastomer in which biologically active species are incorporated within a cured coating. In these coatings, the amount of polymer is relatively high, for example about 70% of the drug-loaded coating (Ding and Helmus, 2002).

While effective in treating deleterious lumen constrictions, vascular stents in an instance of medical irony, also risk re-creating the condition that they were used to treat. Stents can incur the development of thick endothelial tissue inside the lumen--the neointima. Although the degree of development varies, the neointima can grow to occlude the vessel lumen, a type of restenosis. Restenosis is a healing process of damaged coronary arterial walls, with neointimal tissue impinging significantly on the vessel lumen. The toll for temporary passage is therefore steep.

### Rapamycin

A Canadian expedition to Easter Island in 1964 unearthed a fungus that produced a powerful immunosuppressing, anti-fungal, and anti-proliferative molecule. From Easter Island to laboratories in Canada, the fungus landed in Suren Sehgal's hands, who elucidated the properties of a purified compound of the fungus *Streptomyces lygroscopicus* in 1972, but this finding was abandoned. Sehgal resurrected research in 1987 and developed the compound as an immunosuppressant. Today, rapamycin (christened after Rapa Nui, the name by which the Easter Island natives knew their homeland) is used to reduce the risk of organ transplants and the side effects of stents, and is being investigated as a an anti-tumor pharmaceutical.

Rapamycin, also known as sirolimus, is a macrocyclic triene antibiotic that inhibits fungal growth, particularly against *Candida albicans,* both *in vitro* and *in vivo* (Baker *et al.,* 1978; Sehgal, 1975; Sehgal, 1976; Sehgal *et al.,* 1975; Vezina *et al.,* 1975). Rapamycin alone (Surendra, 1989) or in combination with picibanil (Eng, 1983) has been shown to have anti-tumor activity. In 1977, rapamycin was shown to be effective as an immunosuppressant in experimental models for allergic encephalomyelitis (a model for multiple sclerosis), adjuvant arthritis, and rheumatoid arthritis (Martel *et al.,* 1977). Rapamycin also effectively inhibits the formation ot IgE-like antibodies (Martel *et al.,* 1977).

The compound cyclosporine (cyclosporin A) has found wide use since its introduction in the fields of organ transplantation and immunomodulation, and has brought about a significant increase in the success rate for transplantation procedures. Recently, several classes of macrocyclic compounds having potent immunomodulatory activity have been discovered. A number of macrocyclic compounds isolated from the genus *Streptomyces,* including the immunosuppressant FK-506, a 23 membered macrocyclic lactone, which was isolated from a strain *ot S. tsukubaensis* have been previously described (Okuhara et al., 1986).

Other related natural products, including FR-900520 and FR-900523, which differ from FK-506 in their alkyl substituent at C-21, have been isolated from *S. lygroscopicus yakushimnaensis.* Another analog, FR-900525, produced by *S*. *tsukubaensis,* differs from FK-506 in the replacement of a pipecolic acid moiety with a proline group. Unsatisfactory side-effects associated with cyclosporine and FK-506 including nephrotoxicity, have led to a continued search for immunosuppressant compounds having improved efficacy and safety, including an immunosupressive agent which is effective topically, but ineffective systemically (Luly, 1995).

The immunosuppressive effects of rapamycin have also been disclosed in *FASEB,* 1989, *3*, 3411 as has its ability to prolong survival time of organ grafts in histoincompatible rodents (Morris and Meiser, 1989). The ability of rapamycin to inhibit T-cell activation was disclosed by M. Strauch (FASEB, 1989, 3, 3411). These and other biological effects of rapamycin have been previously reviewed (Morris, 1992).

Rapamycin has been shown to reduce neointimal proliferation in animal models, and to reduce the rate of restenosis in humans. Evidence has been published showing that rapamycin also exhibits an anti-inflammatory effect, a characteristic which supported its selection as an agent for the treatment of rheumatoid arthritis. Because both cell proliferation and inflammation are thought to be causative factors in the formation of restenotic lesions after balloon angioplasty and stent placement, rapamycin and analogs thereof have been proposed for the prevention of restenosis.

Ester and diester derivatives of rapamycin (esterification at positions 31 and 42) have been shown to be useful as antifungal agents (Rakhit, 1982) and as water soluble prodrugs of rapamycin (Stella, 1987).

Fermentation and purification of rapamycin and 30-demethoxy rapamycin have been described (Paiva et al., 1991; Sehgal et al., 1983; Sehgal et al., 1975; Vezina et al., 1975).

Numerous chemical modifications of rapamycin have been attempted. These include the preparation of ester and diester derivatives of rapamycin (Caufield, 1992), 27-oximes of rapamycin (Failli, 1992a); 42-oxo analog of rapamycin (Caufield, 1991); bicyclic rapamycins (Kao, 1992a); rapamycin dimers (Kao, 1992b); silyl ethers of rapamycin (Failli, 1992b); and arylsulfonates and sultamates (Failli, 1993). Rapamycin was recently synthesized in its naturally occurring enantiomeric form (Hayward et al., 1993; Nicolaou et al., 1993; Romo et al., 1993).

Rapamycin, like FK-506, binds to FKBP-12 (Bierer *et al*., 1991; Dumont *et al*., 1990; Fretz *et al,* 1991; Harding *et al,* 1989; Siekierka *et al,* 1989). The rapamycin:FKBP-12 complex binds to yet another protein that is distinct from calcineurin, the protein that the FK-506:FKBP-12 complex inhibits (Brown *et al,* 1994; Sabatini *et al,* 1994).

Percutaneous transluminal coronary angioplasty (PTCA) was developed by Andreas Gruntzig in the 1970's. The first canine coronary dilation was performed on September 24, 1975; studies showing the use of PTCA were presented at the annual meetings of the American Heart Association the following year. Shortly thereafter, the first human patient was studied in Zurich, Switzerland, followed by the first American human patients in San Francisco and New York. While this procedure changed the practice of interventional cardiology with respect to treatment of patients with obstructive coronary artery disease, the procedure did not provide long-term solutions. Patients received only temporary abatement of the chest pain associated with vascular occlusion; repeat procedures were often necessary. It was determined that the existence of restenotic lesions severely limited the usefulness of the new procedure. In the late 1980's, stents were introduced to maintain vessel patency after angioplasty. Stenting is involved in 90% of angioplasty performed today. Before the introduction of stents, the rate of restenosis ranged from 30% to 50% of the patients who were treated with balloon angioplasty. The recurrence rate after dilatation of in-stent restenosis may be as high as 70% in selected patient subsets, while the angiographic restenosis rate in de novo stent placement is about 20%. Placement of the stent reduced the restenosis rate to 15% to 20%. This percentage likely represents the best results obtainable with purely mechanical stenting. The restenotic lesion is caused primarily by neointimal hyperplasia, which is distinctly different from atherosclerotic disease both in time-course and in histopathologic appearance. Restenosis is a healing process of damaged coronary arterial walls, with neointimal tissue impinging significantly on the vessel lumen. Vascular brachytherapy appears to be efficacious against in-stent restenotic lesions. Radiation, however, has limitations of practicality and expense, and lingering questions about safety and durability.

Accordingly, it is desired to reduce the rate of restenosis by at least 50% of its current level. It is for this reason that a major effort is underway by the interventional device community to fabricate and evaluate drug-eluting stents. Such devices could have many advantages if they were successful, principally since such systems would need no auxiliary therapies, either in the form of periprocedural techniques or chronic oral pharmacotherapy.

### Zotarolimus

ABT-578 [40-epi-(1-tetrazolyl)-rapamycin], known better today as zotarolimus, is a semi-synthetic macrolide triene antibiotic derived from rapamycin. Zotarolimus is a potent inhibitor of T-cell lymphocyte proliferation, similar to its precursor rapamycin. Zotarolimus has found exceptional applications in coating cardiovascular stents, especially drug-eluting stents (DES's) to minimize restenosis (Mollison *et al.,* 2003). Zotarolimus structure is shown in Scheme I.

Other chemical modifications of rapamycin have been attempted. These include the preparation of mono- and di-ester derivatives of rapamycin (Caufield, 1992), 27-oximes of rapamycin (Failli, 1992a); 42-oxo analog of rapamycin (Caufield, 1991); bicyclic rapamycin (Kao, 1992a); rapamycin dimers (Kao, 1992b); silyl ethers of rapamycin (Failli, 1992b); and arylsulfonates and sultamates (Failli, 1993).

In addition to its anti-fungal, immunosuppressant and anti-tumor activities, rapamycin and zotarolimus, like other mTOR inhibitors, reduce neointimal proliferation in animal models, as well as the rate of restenosis in humans. Rapamycin and zotarolimus also exhibit anti-inflammatory effects. Stents coated with analogues of rapamycin, including tacrolimus (FK506), rapamycin, everolimus and especially zotarolimus, are effective at preventing restenosis in clinical trials.

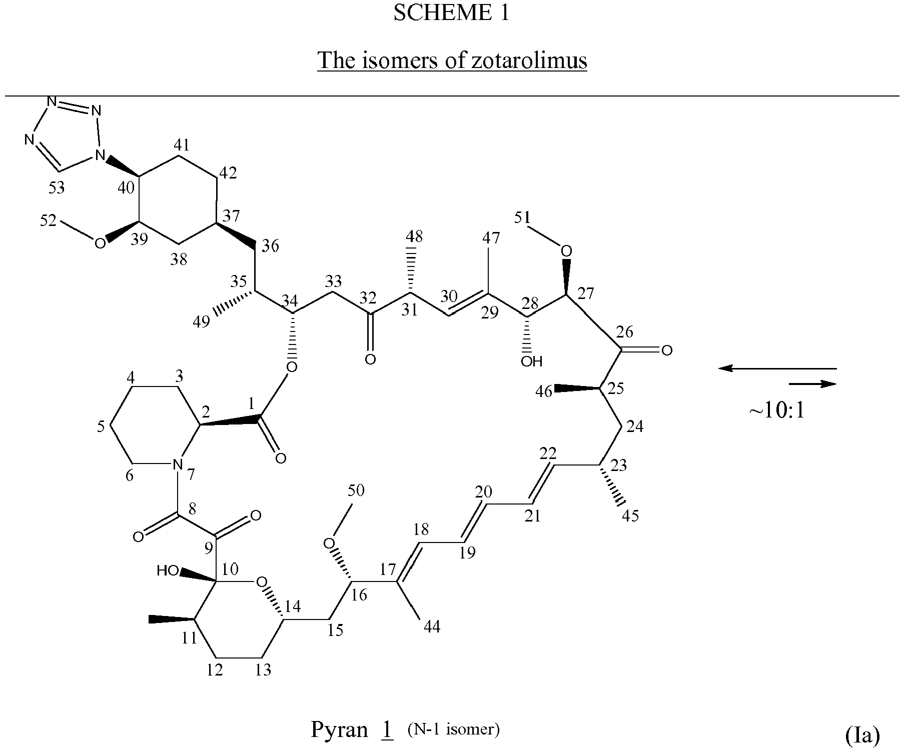

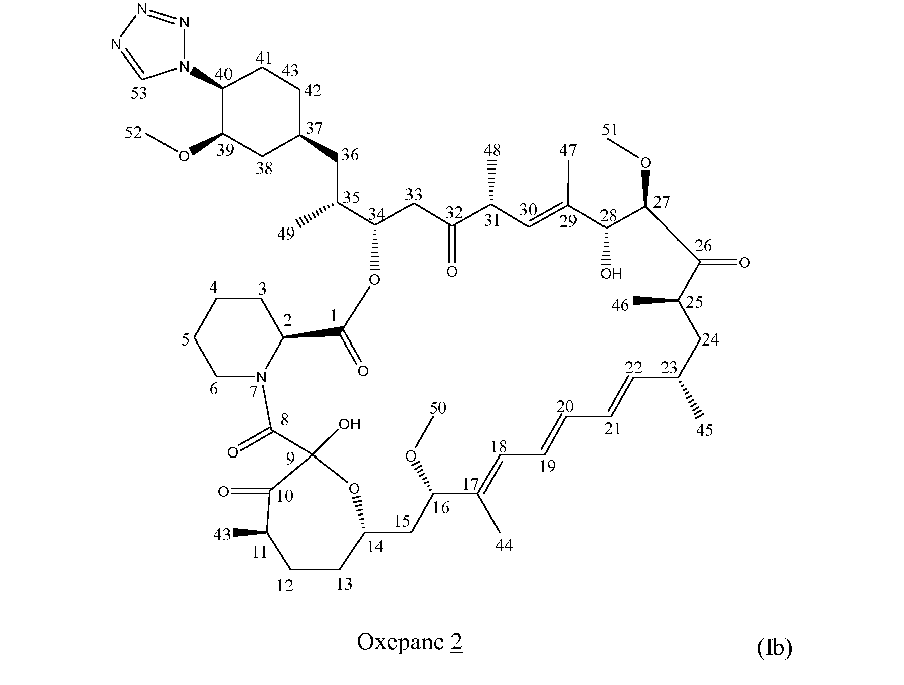

### Brief Description of the Drawings

**Figure 1A** shows a side view of an example of a stent.
**Figure 1B** shows blood concentrations ± SEM (n=3) of tetrazole-containing rapamycin analogs dosed in monkey.
**Figure 2** is a side view in elevation showing an example stent.
**Figure 3A** is a cross-sectional view of a vessel segment in which was placed a stent coated with a polymer only.
**Figure 3B** is a cross-sectional view of a vessel segment in which was placed a stent coated with a polymer plus drug.
**Figure 4** shows *in vitro* elution profiles of stainless steel stents containing zotarolimus 10 µg/mm and covered with PC topcoats of 0, 2, 5, and 10 µg/mm (n=12 stents per time point; mean ± SEM). Note the slower elution rate as topcoat thickness increases."
**Figure 5** shows the neointimal areas (30% overstretch) after 28 days of implantation in swine blood vessels of drug-eluting and non-drug eluting stents; boxed numbers indicate the number of stents per group.
**Figure 6** shows neointimal thicknesses (30% overstretch) after 28 days of implantation in swine blood vessels of drug-eluting and non-drug eluting; boxed numbers indicate the number of stents per group.
**Figure 7** shows percent area stenoses (30% overstretch) after 28 days of implantation in swine blood vessels of drug-eluting and non-drug-eluting stents; boxed numbers indicate the number of stents per group.
**Figures 8A-D** show micrographs of cross-sections of representative blood vessels from a swine study, representing average neointimal areas for each group. Figure 19A, TriMaxx™, stent; 19B, ZoMaxx™, stent; 19C, Cypher® stent; 19D, Taxus® stent.
**Figure 9** shows a graph of the results of a 28-day elution study to assess the tissue distribution of zotarolimus from polymer coated stents (ZoMaxx™; filled circles) in rabbit iliac arteries and compared to stents coated with rapamycin (Cypher®; filled squares); these data represent the amount of drug eluted from the implanted stents at the indicated time points.
**Figure 10** shows a graph of the results of a 28-day elution study to assess the tissue distribution of zotarolimus from polymer coated stents (ZoMaxx™; filled circles) in rabbit iliac arteries and compared to stents coated with rapamycin (Cypher®; filled squares); these data represent the amount of drug present in tissue adjacent to the implanted stents at the indicated time points.
**Figure 11** shows a graph of the arterial concentrations of zotarolimus and rapamycin after ZoMaxx™ or Cypher® stenting in rabbits and pigs. Filled squares, ZoMaxxTM/pig; filled triangles, Cypher®/pig; upside-down filled triangle, ZoMaxxTM/rabbit; side-ways filled triangle, Cypher®/rabbit.
**Figure 12** shows a graph of the results of a 28-day elution study to assess the tissue distribution of zotarolimus from polymer coated stents (ZoMaxx™; filled circles) in rabbit iliac arteries and compared to stents coated with rapamycin (Cypher®; filled squares); these data represent the blood concentration of each drug at the indicated time points.
**Figure 13** shows a graph of the results of long-term implantation of ZoMaxx^{™} (large filled squares) or Cypher® (smaller filled squares) stents in a pig model. Differences that are statistically significant (p<0.05) are indicated by *. Cypher® data from (Carter *et al.,* 2004).
**Figure 14** shows a graph of the results of long-term inflammation scores in the porcine overstretch model for zotarolimus polymer coated stents (ZoMaxx™; filled triangles) compared to stents coated with rapamycin (Cypher®; filled diamonds). Cypher® data from (Carter *et al.,* 2004).

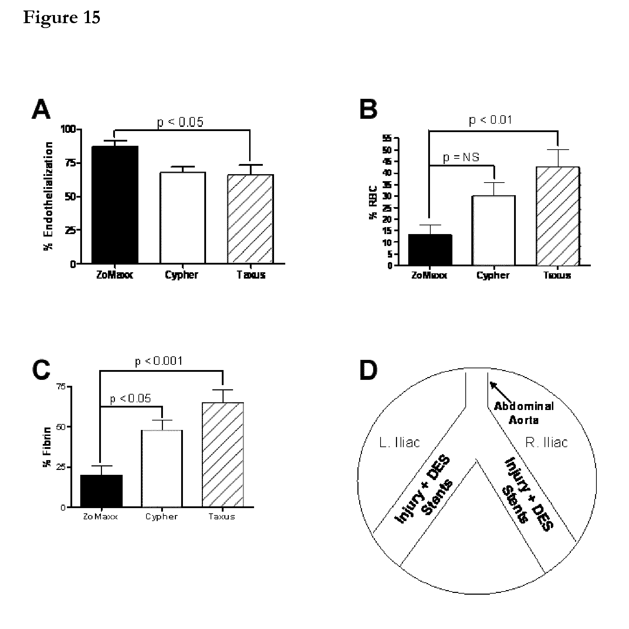

**Figure 15A-D** shows the results of a study of stent implantation in a rabbit overlap model at 28 days. Three assays indicative of inflammation and efficacy are shown. Figure 15A shows the percentage of the implanted stents that are coated with endothelial cells; Figure 15B shows the percent of red blood cells in the stent area; and Figure 15C shows the percent of fibrin in the stent area. In each case, statistically significant differences are indicated by brackets and the p-value threshold for significance. In each graph, solid bars represent ZoMaxx^{™}, white bars represent Cypher®, and hatched bars represent Taxus stents, coated with paclitaxel. Figure 15D shows the sites of stent implantation in the iliac arteries.

### Summary of the Invention

In a first aspect, the invention is drawn to drug delivery systems that have a supporting structure, which has a pharmaceutically acceptable carrier or excipient; and a first therapeutic composition comprising zotarolimus or prodrugs, derivatives, esters, salts thereof, wherein, when the systems are implanted in a body lumen of a subject, delivery of zotarolimus to a lumen wall adjacent to the system is greater than that when compared to delivery of a control therapeutic composition from a control drug delivery system containing a similar dose to the first therapeutic composition. The control therapeutic can be an olimus drug, including everolimus, rapamycin, tacrolimus (FK506), biolimus A9, CCI-779, RAD 001, AP23573 and combinations thereof; as well as an anti-inflammatory, including dexamethasone hydrocortisone, estradiol, acetaminophen, ibuprofen, naproxen, fluticasone, clobetasol, adalimumab, sulindac, and combinations thereof. Both systems can include addition therapeutics, such as other olimus drugs and anti-inflammatories, as well as anti-proliferative agents, anti-platelet agents, anti-thrombolytic and anti-thrombotic agents, including antibodies. In such systems, zotarolimus delivery to the lumen wall can be increased for at least 28 days after implantation when compared to the control. Furthermore, the cumulative percentage of zotarolimus eluted from the system can be significantly greater than a cumulative percentage of rapamycin eluted from the control drug delivery system containing rapamycin 28 days after implantation. The difference of drug delivery can be striking, from 5-fold greater than that of the control therapeutic 14 days or less after implantation of the systems up to, and even exceeding 10-fold greater than that of the control therapeutic. The body lumen can be, for example, a blood vessel, in which case, implantation of the system having zotarolimus correlates with a reduction of neointima hyperplasia when compared to the control drug delivery system containing the second therapeutic composition at greater than or equal to three months after implantation. Reduction can be ≥ 60% when compared to the control drug delivery system 180 days after implantation, and ≥ 30% when compared to the control drug delivery system 90 days after implantation. Also, inflammation can be significantly reduced when compared to the control drug delivery system containing a second therapeutic by at least 56 days after implantation, and 182 days after implantation. When used in stent overlap studies, endothelialization is significantly favored when compared to control drug delivery system containing rapamycin 28 days after implantation. Likewise, fibrin production is significantly reduced when compared to control drug delivery system containing rapamycin 28 days after implantation in stent overlap studies. When the systems can include For example, such stents can have a concentration of zotarolimus is 10 µg/mm of the stent, and the concentration of the control therapeutic composition is 10 µg/mm of the stent; and the control therapeutic can include rapamycin. Subjects that can benefit from such systems include vertebrates, such as pigs, rabbits, and humans.

In a second aspect, the invention is drawn to drug delivery systems a supporting structure having a pharmaceutically acceptable carrier or excipient; and a first therapeutic composition comprising zotarolimus or prodrugs, derivatives, esters, salts thereof, wherein, when the system is implanted in a body lumen of a subject, neointima hyperplasia is significantly reduced when compared to delivery of a control therapeutic composition from a control drug delivery system containing a similar dose to the first therapeutic composition at 90 days or greater after implantation. The control therapeutic can be an olimus drug, including everolimus, rapamycin, tacrolimus (FK506), biolimus A9, CCI-779, RAD 001, AP23573 and combinations thereof; as well as an anti-inflammatory, including dexamethasone hydrocortisone, estradiol, acetaminophen, ibuprofen, naproxen, fluticasone, clobetasol, adalimumab, sulindac, and combinations thereof. Both systems can include addition therapeutics, such as other olimus drugs and anti-inflammatories, as well as anti-proliferative agents, anti-platelet agents, anti-thrombolytic and anti-thrombotic agents, including antibodies. In such systems, zotarolimus delivery to the lumen wall can be increased for at least 28 days after implantation when compared to the control. Furthermore, the cumulative percentage of zotarolimus eluted from the system can be significantly greater than a cumulative percentage of rapamycin eluted from the control drug delivery system containing rapamycin 28 days after implantation. The difference of drug delivery can be striking, from 5-fold greater than that of the control therapeutic 14 days or less after implantation of the systems up to, and even exceeding 10-fold greater than that of the control therapeutic. The body lumen can be, for example, a blood vessel, in which case, implantation of the system having zotarolimus correlates with a reduction of neointima hyperplasia when compared to the control drug delivery system containing the second therapeutic composition at greater than or equal to three months after implantation. Reduction can be ≥ 60% when compared to the control drug delivery system 180 days after implantation, and ≥ 30% when compared to the control drug delivery system 90 days after implantation. Also, inflammation can be significantly reduced when compared to the control drug delivery system containing a second therapeutic by at least 56 days after implantation, and 182 days after implantation. When used in stent overlap studies, endothelialization is significantly favored when compared to control drug delivery system containing rapamycin 28 days after implantation. Likewise, fibrin production is significantly reduced when compared to control drug delivery system containing rapamycin 28 days after implantation in stent overlap studies. When the systems can include For example, such stents can have a concentration of zotarolimus is 10 µg/mm of the stent, and the concentration of the control therapeutic composition is 10 µg/mm of the stent; and the control therapeutic can include rapamycin. Subjects that can benefit from such systems include vertebrates, such as pigs, rabbits, and humans.

In a third aspect, the invention is drawn to drug delivery systems that have a supporting structure having a pharmaceutically acceptable carrier or excipient; and a therapeutic composition comprising zotarolimus or prodrugs, derivatives, esters, or salts thereof, wherein, when the system is implanted in a body lumen of a subject, inflammation is significantly reduced when compared to delivery of a control therapeutic composition from a control drug delivery system containing a similar dose to the first therapeutic composition at 90 days after implantation. The control therapeutic can be an olimus drug, including everolimus, rapamycin, tacrolimus (FK506), biolimus A9, CCI-779, RAD 001, AP23573 and combinations thereof; as well as an anti-inflammatory, including dexamethasone hydrocortisone, estradiol, acetaminophen, ibuprofen, naproxen, fluticasone, clobetasol, adalimumab, sulindac, and combinations thereof. Both systems can include addition therapeutics, such as other olimus drugs and anti-inflammatories, as well as anti-proliferative agents, anti-platelet agents, anti-thrombolytic and anti-thrombotic agents, including antibodies. In such systems, zotarolimus delivery to the lumen wall can be increased for at least 28 days after implantation when compared to the control. Furthermore, the cumulative percentage of zotarolimus eluted from the system can be significantly greater than a cumulative percentage of rapamycin eluted from the control drug delivery system containing rapamycin 28 days after implantation. The difference of drug delivery can be striking, from 5-fold greater than that of the control therapeutic 14 days or less after implantation of the systems up to, and even exceeding 10-fold greater than that of the control therapeutic. The body lumen can be, for example, a blood vessel, in which case, implantation of the system having zotarolimus correlates with a reduction of neointima hyperplasia when compared to the control drug delivery system containing the second therapeutic composition at greater than or equal to three months after implantation. Reduction can be ≥ 60% when compared to the control drug delivery system 180 days after implantation, and ≥ 30% when compared to the control drug delivery system 90 days after implantation. Also, inflammation can be significantly reduced when compared to the control drug delivery system containing a second therapeutic by at least 56 days after implantation, and 182 days after implantation. When used in stent overlap studies, endothelialization is significantly favored when compared to control drug delivery system containing rapamycin 28 days after implantation. Likewise, fibrin production is significantly reduced when compared to control drug delivery system containing rapamycin 28 days after implantation in stent overlap studies. When the systems can include For example, such stents can have a concentration of zotarolimus is 10 µg/mm of the stent, and the concentration of the control therapeutic composition is 10 µg/mm of the stent; and the control therapeutic can include rapamycin. Subjects that can benefit from such systems include vertebrates, such as pigs, rabbits, and humans.

In yet another aspect, the invention is drawn to treating subjects using the drug delivery systems of the invention, and include implanting the systems in blood vessel lumens.

In an additional aspect, the invention is directed to drug delivery systems that have a supporting structure capable of having a pharmaceutically acceptable carrier or excipient; and a therapeutic composition that includes zotarolimus or prodrugs, derivatives, esters, or salts thereof, wherein zotarolimus is significantly eluted from the supporting structure 30 days after implantation in a lumen of a blood vessel of a subject. The eluted zotarolimus can comprise 85% to 100% of the zotarolimus loaded onto the device 15 - 30 days after implanting the device, and the eluted zotarolimus is 5- to 15-fold more concentrated in walls of the blood vessel adjacent to the delivery system when compared to a control drug delivery system containing rapamycin. If, for example, the control therapeutic composition is rapamycin, then the amount of zotarolimus in tissue is greater than rapamycin at the same time point; and likewise, the concentration of zotarolimus in blood is less than the concentration of rapamycin at the same time point. In this system, a concentration cₑ of eluted zotarolimus per unit of blood vessel wall adjacent to the delivery system is at time t in hours after implantation includes:
when 0 ≤ t < 120, then 6 µg/g ≤ cₑ ≤ 113 µg/g;
when 120 ≤ t < 168, then 5 µg/g ≤ cₑ ≤ 40 µg/g; and
when 168 ≤ t < 720, then 2.5 µg/g ≤ cₑ ≤ 50 µg/g. And likewise, whole blood concentration, c_{b}, of zotarolimus per ml of blood is at day d after implantation in a rabbit comprises:
   when 0 ≤ d ≤ 2, then 1.5 ≤ c_{b} ≤ 4;
   when 2 < d ≤ 3, then 1.4 ≤ c_{b} ≤ 1.5;
   when 3 < d ≤ 4, then 1.3 ≤ c_{b} ≤ 1.4; and
   when 4 < d ≤ 28, then 0 ≤ c_{b} ≤ 1.3. The neointimal area of the blood vessel lumen implanted with the system is significantly less than the neotinimal area of the blood vessel lumen implanted with the control system at greater than or equal to 90 days; likewise, the neointimal area of the blood vessel lumen implanted with the system is less than or equal to 1.5 mm² 30 days or more after implantation in an over-stretch study. The system also has significantly reduced inflammation 90 days or more after implantation of the system when compared to the control system. Also noteworthy, endothelial cells covering a surface of the systems are significantly confluent 28 days after implantation of the system in an overlap rabbit model, such endothelialization exceeding 75%.

### Detailed Description of the Invention

The invention provides systems for drug delivery and methods of treatments using the systems. The systems of the invention exploit the combination of zotarolimus combined with a solid support and optionally, a polymer coating. These systems have exceptional properties, especially when compared to currently available products, including stents coated with rapamycin or tacrolimus (FK506). These advantages include increased drug delivery to tissues immediately adjacent to the systems, as opposed to systemic distribution of the drug, decreased inflammation, and most importantly, long-term efficacy. Thus the systems of the invention better avoid the instances of medical irony , wherein the condition treated returns as a side-effect of the treatment, as is especially the case when the system is implanted in a blood vessel lumen to keep it open.

### Definitions

**Prodrug** refers to compounds which are rapidly transformed *in vivo* to the parent compound of the above formula, for example, by hydrolysis in blood. A thorough discussion is provided by Higuchi and V. Stella (Higuchi and Stella, 1987) and by Roche (Roche, 1987).

**Pharmaceutically acceptable prodrugs** refers to those prodrugs of pharmaceuticals which are, within the scope of sound medical judgment, suitable for use in contact with the tissues of humans and lower mammals without undue toxicity, irritation, and allergic response, are commensurate with a reasonable benefit/risk ratio, and are effective for their intended use, as well as the zwitterionic forms, where possible, of the compounds of the invention. Particularly useful pharmaceutically acceptable prodrugs of this invention are prodrug esters of the C-31 hydroxyl group of compounds of this invention.

**Olimus** drugs include everolimus, rapamycin, tacrolimus (FK506), biolimus A9, CCI-779, RAD 001, and AP23573.

**Prodrug esters** refers to any of several ester-forming groups that are hydrolyzed under physiological conditions. Examples of prodrug ester groups include acetyl, ethanoyl, pivaloyl, pivaloyloxymethyl, acetoxymethyl, phthalidyl, methoxymethyl, and indanyl, as well as ester groups derived from the coupling of naturally or unnaturally-occurring amino acids to the C-31 hydroxyl group of compounds of this invention.

**Significantly** refers to a difference that is statistically significant.

**Subject** means a vertebrate, including a warm-blooded vertebrate, including a monkey, dog, cat, rabbit, cow, pig, goat, sheep, horse, rat, mouse, guinea pig, *etc*.; and a human.

**Supporting structure** means a framework that is capable of containing or supporting a pharmaceutically acceptable carrier or excipient, which carrier or excipient can have one or more therapeutic agents or substances, *e*.*g*., one or more drugs and/or other compounds. The supporting structure is typically formed of metal or a polymeric material. Suitable supporting structures formed of polymeric materials, including biodegradable polymers, capable of including the therapeutic agents or substances include, without limitation, those disclosed in U.S. Patent Nos. 6,413,272 and 5,527,337 (Igaki, 2002; Stack *et al.,* 1996).

**Therapeutic compound** means any pharmaceutical substance that when administered to a subject appropriately at an appropriate doses, has a beneficial effect on the subject.

In the following sections, first the polymers that can be used with the systems of the invention of zotarolimus are discussed, followed by discussions of the pharmaceutical compositions. Following that, a discussion of drug combinations that can be administered with the systems are also outlined, and then methods of treatment, including a listing of some of the many diseases and conditions that can benefit from administration of the systems of the invention. Afterwards, several tests are presented to allow one of skill in the art to ascertain the safety, efficacy and kinetics of drug release for the systems of the invention. The final section presents examples that illustrate and support various aspects of the invention.

### Polymers

When used in the invention, the coating can comprise any polymeric material in which the therapeutic agent, *i.e.,* the drug, is substantially soluble, or can be effectively dispersed. The purpose of the coating is to serve as a controlled release vehicle for the therapeutic agent or as a reservoir for a therapeutic agent to be delivered at the site of a lesion. The coating can be polymeric and can further be hydrophilic, hydrophobic, biodegradable, or non-biodegradable. The material for the polymeric coating can be selected from the group consisting of polycarboxylic acids, cellulosic polymers, gelatin, polyvinylpyrrolidone, maleic anhydride polymers, polyamides, polyvinyl alcohols, polyethylene oxides, glycosaminoglycans, polysaccharides, polyesters, polyurethanes, silicones, polyorthoesters, polyanhydrides, polycarbonates, polypropylenes, polylactic acids, polyglycolic acids, polycaprolactones, polyhydroxybutyrate valerates, polyacrylamides, polyethers, and mixtures and copolymers of the foregoing. Coatings prepared from polymeric dispersions including polyurethane dispersions (BAYHYDROL, *etc.*) and acrylic acid latex dispersions can also be used with the therapeutic agents.

Biodegradable polymers include polymers including poly(L-lactic acid), poly(DL-lactic acid), polycaprolactone, poly(hydroxy butyrate), polyglycolide, poly(diaxanone), poly(hydroxy valerate), polyorthoester; copolymers including poly (lactide-co-glycolide), polyhydroxy (butyrate-co-valerate), polyglycolide-co-trimethylene carbonate; polyanhydrides; polyphosphoester; polyphosphoester-urethane; polyamino acids; polycyanoacrylates; biomolecules including fibrin, fibrinogen, cellulose, starch, collagen and hyaluronic acid; and mixtures of the foregoing. Biostable materials that are suitable for use in this invention include polymers including polyurethane, silicones, polyesters, polyolefins, polyamides, polycaprolactam, polyimide, polyvinyl chloride, polyvinyl methyl ether, polyvinyl alcohol, acrylic polymers and copolymers, polyacrylonitrile, polystyrene copolymers of vinyl monomers with olefins (including styrene acrylonitrile copolymers, ethylene methyl methacrylate copolymers, ethylene vinyl acetate), polyethers, rayons, cellulosics (including cellulose acetate, cellulose nitrate, cellulose propionate, *etc.*), parylene and derivatives thereof; and mixtures and copolymers of the foregoing.

Another useful polymer is poly(MPC_{w}:LMAYₓ:HPMA_{y}:TSMA_{z}) where w, x, y, and z represent the molar ratios of monomers used in the feed for preparing the polymer and MPC represents the unit 2-methacryoyloxyethylphosphorylcholine, LMA represents the unit lauryl methacrylate, HPMA represents the unit 2-hydroxypropyl methacrylate, and TSMA represents the unit 3-trimethoxysilylpropyl methacrylate. The drug-impregnated stent can be used to maintain patency of a coronary artery previously occluded by thrombus, atherosclerotic plaque, and/or proliferative smooth muscle cells. The delivery of an anti-proliferative agent reduces the rate ot in-stent restenosis.

The compounds or drugs described herein can be applied to stents that have been coated with a polymeric compound, including those previously described (Bowers et al., 2000; Bowers et al., 1998; Lewis and Leppard, 2002; Lewis and Leppard, 2005). Incorporation of the compound or drug into the polymeric coating of the stent can be carried out by dipping the polymer-coated stent into a solution having the compound or drug for a sufficient period of time (including, for example, five minutes) and then drying the coated stent, by means of air drying for a sufficient period of time (including, for example, 30 minutes). Other methods of applying therapeutic compounds, including spraying or ink-jet application, can be used. The polymer-coated stent having the compound or drug can then be delivered to the coronary vessel by deployment from a balloon catheter. In addition to stents, other devices that can be used to introduce the drugs of this invention to the vasculature include, but are not limited to grafts, catheters, and balloons.

Overcoat thickness (if an overcoat is used) can be used to modulate drug delivery without excessively impeding release kinetics of the drugs.

### Polymer layers and therapeutic compounds on medical devices

There is much flexibility in providing suitable drug-loaded polymer layers to be included on medical devices, such as a stent (*e.g.,* **Figure 1A**). For example, within therapeutic window parameters (generally levels between therapeutically effective and toxicity) associated with the drugs of interest, ratios of any drugs used in combination can be varied relative to each other. For example, an embodiment has a 90:10 total drug:polymer ratio where the ratio of drugs in the combination can be 1:1. Thus, a stent delivering a zotarolimus/paclitaxel combination can have 10 µg/mm zotarolimus and 10 µg/mm paclitaxel in a PC polymer layer with a 5 µg/mm PC topcoat. Total drug:polymer ratio can be lower, however, *e*.*g*., 40:60 or less. Upper limits on the total amount of drug will depend on several factors, including miscibility of the selected drugs in the selected polymer, the stability of the drug/polymer mixture, *e*.*g*., compatibility with sterilization, and the physical properties of the mixture, *e*.*g*., flowability/processability, elasticity, brittleness, viscosity (including this coating does not web or bridge between stent struts), coating thickness that adds substantially to the stent profile or causes delamination or cracking or is difficult to crimp. Typical stent struts are spaced about 60-80 microns apart, suggesting an upper limit of the drug/polymer/polymer overcoat is about 30 microns.

### Pharmaceutical compositions

Pharmaceutical compositions comprise at least one therapeutic compound and a pharmaceutically acceptable carrier or excipient, which can be administered orally, rectally, parenterally, intracisternally, intravaginally, intraperitoneally, topically (as by powders, ointments, drops or transdermal patch), bucally, as an oral or nasal spray, or locally, as in a stent placed within the vasculature. Pharmaceutically acceptable carriers are non-toxic solid, semi-solid or liquid filler, diluent, encapsulating materials or formulations auxiliary of any type. Parenteral administration includes intravenous, intraarterial, intramuscular, intraperitoneal, intrasternal, intrathecal or intraspinal, subcutaneous or intradermal, and intraarticular injection, infusion, and placement, including, for example, in vasculature.

Pharmaceutical compositions for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (including glycerol, propylene glycol, and polyethylene glycol), carboxymethylcellulose and suitable mixtures thereof, vegetable oils (including olive oil), and injectable organic esters including ethyl oleate. Proper fluidity can be maintained, for example, by the use of coating materials including lecithin, by the maintenance of the required particle size in the case of dispersions, and by the use of surfactants.

These compositions can also include adjuvants including preservatives, wetting agents, emulsifying agents, and dispersing agents. Prevention of the action of microorganisms can be ensured by the inclusion of various antibacterial and antifungal agents, for example, paraben, chlorobutanol, and phenol sorbic acid. It can also be desirable to include isotonic agents including sugars, and sodium chloride. Prolonged absorption of the injectable pharmaceutical form can be brought about by the inclusion of agents that delay absorption including aluminum monostearate and gelatin.

To prolong the effect of the drug, the absorption of the drug can be retarded by subcutaneous or intramuscular injection. This can be accomplished by the use of a liquid suspension of crystalline or amorphous material with poor water solubility. The rate of absorption of the drug then depends upon its rate of dissolution which, in turn, can depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers including polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier including sodium citrate or dicalcium phosphate and/or (a) fillers or extenders including starches, lactose, sucrose, glucose, mannitol, and silicic acid, (b) binders including, for example, carboxymethylcellulose, alginates, gelatin, polyvinylpyrrolidone, sucrose, and acacia, (c) humectants including glycerol, (d) disintegrating agents including agar-agar, calcium carbonate, potato or tapioca starch, alginic acid, certain silicates, and sodium carbonate, (e) solution retarding agents including paraffin, (f) absorption accelerators including quaternary ammonium compounds, (g) wetting agents including, for example, cetyl alcohol and glycerol monostearate, (h) absorbents including kaolin and bentonite clay, and (i) lubricants including talc, calcium stearate, magnesium stearate, solid polyethylene glycols, sodium lauryl sulfate, and mixtures thereof. In the case of capsules, tablets and pills, the dosage form can also comprise buffering agents.

Solid compositions of a similar type can also be employed as fillers in soft, semi-solid and hard-filled gelatin capsules or liquid-filled capsules using such excipients as lactose or milk sugar as well as high molecular weight polyethylene glycols.

The solid dosage forms of tablets, dragees, capsules, pills, and granules can be prepared with coatings and shells including enteric coatings and other coatings well known in the pharmaceutical formulating art. They can optionally include opacifying agents and can also be of a composition that they release the active ingredient(s) only, or preferentially, in a certain part of the intestinal tract, optionally, in a delayed manner. Examples of embedding compositions that can be used include polymeric substances and waxes. Those embedding compositions having a drug can be placed on medical devices, including stents, grafts, catheters, and balloons.

The active compounds can also be in micro-encapsulated form, if appropriate, with one or more excipients.

Compositions for rectal or vaginal administration are suppositories or retention enemas which can be prepared by mixing the compounds of this invention with suitable non-irritating excipients or carriers including cocoa butter, polyethylene glycol or a suppository wax which are solid at room temperature but liquid at body temperature and therefore melt in the rectum or vaginal cavity and release the active compound.

### Drug combinations

The compounds described herein for use in polymer-coated stents can be used in combination with other pharmacological agents. The pharmacological agents that would, in combination with the compounds of this invention, be most effective in preventing restenosis can be classified into the categories of anti-proliferative agents, anti-platelet agents, anti-inflammatory agents, anti-thrombotic agents, and thrombolytic agents. These classes can be further sub-divided. For example, anti-proliferative agents can be anti-mitotic. Anti-mitotic agents inhibit or affect cell division, whereby processes normally involved in cell division do not take place. One sub-class of anti-mitotic agents includes vinca alkaloids. Representative examples of vinca alkaloids include, but are not limited to, vincristine, vinblastine, paclitaxel, etoposide, teniposide, nocodazole, indirubin, and anthracycline derivatives, including, for example, daunorubicin, daunomycin, and plicamycin. Other sub-classes of anti-mitotic agents include anti-mitotic alkylating agents, including, for example, cyclophosphamide, cisplatin, carmustine, tauromustine, bofumustine, and fotemustine, and anti-mitotic metabolites, including, for example, methotrexate, fluorouracil, 5-bromodeoxyuridine, 6-azacytidine, and cytarabine. Anti-mitotic alkylating agents affect cell division by covalently modifying DNA, RNA, or proteins, thereby inhibiting DNA replication, RNA transcription, RNA translation, protein synthesis, or combinations of the foregoing. Other antiproliferative or anti-neoplastic agents which may be utilized include anti-biotics including bleomycin or plicamycin. Antiproliferative agents which act as inhibitors of protein kinase may also be utilized. These include Y-27632, AP23464, PD98059, genistein, staurosporine, PKC412, CGP-41251, daphnetin, SB203580, KN-62, H-7, TKI963, RPR101511A and K252A.

An exemplary anti-mitotic is paclitaxel. As used herein, paclitaxel includes the alkaloid itself and naturally occurring forms and derivatives thereof, as well as synthetic and semi-synthetic forms thereof.

Anti-platelet agents are therapeutic entities that act by (1) inhibiting adhesion of platelets to a surface, typically a thrombogenic surface, (2) inhibiting aggregation of platelets, (3) inhibiting activation of platelets, or (4) combinations of the foregoing. Activation of platelets is a process whereby platelets are converted from a quiescent, resting state to one in which platelets undergo a number of morphologic changes induced by contact with a thrombogenic surface. These changes include changes in the shape of the platelets, accompanied by the formation of pseudopods, binding to membrane receptors, and secretion of small molecules and proteins, including, for example, ADP and platelet factor 4. Anti-platelet agents that act as inhibitors of adhesion of platelets include, but are not limited to, eptifibatide, tirofiban, RGD (Arg-Gly-Asp)-based peptides that inhibit binding to glycoprotein IIbIIIa or αvβ3, antibodies that block binding to glycoprotein IIaIIIb or αvβ3, anti-P-selectin antibodies, anti-E-selectin antibodies, peptides that block P-selectin or E-selectin binding to their respective ligands, saratin, and anti-von Willebrand factor antibodies. Agents that inhibit ADP-mediated platelet aggregation include, but are not limited to, disagregin and cilostazol. Other anti-platelet agents that may be utilized include clopidogrel, dipyridamole and ticlopidine.

Anti-inflammatory agents can also be used. Examples of these include, but are not limited to, prednisone, dexamethasone, hydrocortisone, estradiol, and non-steroidal antiinflammatories, including, for example, the salicylic acid derivatives aspirin, sodium salicylate, salsalate, diflunisal, salicylsalicylic acid, sulfasalazine and olsalazine, the para-aminophenol derivatives including acetaminophen, the arylpropionic acids including ibuprofen, naproxen, ketoprofen, flurbiprofen, fenoprofen and oxaprozin, the anthranilic acids including mefanamic acid and meclofenamic acid, the heteroaryl acetic acids including tolmetin, diclofenac and ketorolac, the enolic acids including oxicams (piroxicam, tenoxicam, meloxicam), pyrazolidinediones (phenylbutazone), the indole and indene acetic acids including indomethacin, sulindac and etodolac and the alkanones (nabumetone). Additional antiinflammatory agents that may be utilized include leflunomide, fluticasone, clobetasol and adalimumab. Other examples of anti-inflammtory agents include those that inhibit binding of cytohines or chemohines to the cognate receptors to inhibit pro-inflammatory signals transduced by the cytokines or the chemokines. Representative examples of these agents include, but are not limited to, anti-IL1, anti-IL2, anti-IL3, anti-IL4, anti-IL8, anti-IL15, anti-MCP1, anti-GM-CSF, and anti-TNF antibodies.

Anti-thrombotic agents include chemical and biological entities that can intervene at any stage in the coagulation pathway. Examples of specific entities include, but are not limited to, small molecules that inhibit the activity of factor Xa. In addition, heparinoid-type agents that can inhibit both FXa and thrombin, either directly or indirectly, including, for example, heparin, heparan sulfate, low molecular weight heparins, including, for example, the compound having the trademark Clivarin®, and synthetic oligosaccharides, including, for example, the compound having the trademark Arixtra®. Other inhibitors of Factor Xa include fondaparinux and idraparinux. Also included are direct thrombin inhibitors, including, for example, melagatran, ximelagatran, argatroban, inogatran, and peptidomimetics of binding site of the Phe-Pro-Arg fibrinogen substrate for thrombin. Additional thrombin inhibitors which may be utilized are hirudin, hirugen, hirulog and bivalrudin. Another class of anti-thrombotic agents that can be delivered are factor VII/VIIa inhibitors, including, for example, anti-factor VII/VIIa antibodies, rNAPc2, and tissue factor pathway inhibitor (TFPI). Additional approaches may include inhibitors of factor Va/VIIIa, including protein C, activated protein C and soluble thrombomodulin and agents which enhance endogenous fibrinolytic activity, including inhibitors of plasminogen activator inhibitor-1 (PAI-1), activated TAFI (TAFIa) or Factor XIIIa.

Thrombolytic agents, which can be defined as agents that help degrade thrombi (clots), can also be used as adjunctive agents, because the action of lysing a clot helps to disperse platelets trapped within the fibrin matrix of a thrombus. Representative examples of thrombolytic agents include, but are not limited to, urokinase or recombinant urokinase, pro-urokinase or recombinant pro-urokinase, tissue plasminogen activator or its recombinant form, alteplase, anistreplase, retaplase and streptokinase.

Other drugs that can be used in combination with the compounds of this invention are cytotoxic drugs, including, for example, apoptosis inducers, including TGF, and topoisomerase inhibitors, including, 10-hydroxycamptothecin, irinotecan, and doxorubicin. Other classes of drugs that can be used in combination with the compounds of this invention are drugs that inhibit cell de-differentiation and cytostatic drugs.

Other agents that can be used in combination with the compounds of this invention include anti-lipaedemic agents, including, for example, fenofibrate, matrix metalloproteinase inhibitors, including, for example, batimistat, antagonists of the endothelin-A receptor, including, for example, darusentan, and antagonists of the αvβ3 integrin receptor.

Zotarolimus compounds and its derivatives can also be co-administered with one or more immunosuppressant agents. The immunosuppressant agents within the scope of this invention include, but are not limited to, IMURAN® azathioprine sodium, brequinar sodium, SPANIDIN® gusperimus trihydrochloride (also known as deoxyspergualin), mizoribine (also known as bredinin), CELLCEPT® mycophenolate mofetil, NEORAL® Cylosporin A (also marketed as different formulation of Cyclosporin A under the trademark SANDIMMUNE®), PROGRAF® tacrolimus (also known as FK-506), rapamycin and RAPAMUNE®, leflunomide (also known as HWA-486), glucocorticoids, including prednisolone and its derivatives, antibody therapies including orthoclone (OKT3) and Zenapax®, and antithymyocyte globulins, including thymoglobulins.

### Co-administration using a stent

When another therapeutic compound is co-administered with zotarolimus using a stent implanted in a vessel, the ratio, *r*, of zotarolimus:compound by weight is such that the activity of one drug does not attenuate the activity of the other (*i.e.,* interfere), and the overall effect of the co-administration is additive, and sometimes synergistic. Useful ratios of zotarolimus:compound in the case where the compound is paclitaxel are greater than approximately 10:7, more approximately 10:7 ≤ *r* ≤ 10:0.01, approximately 10:7 ≤ *r* ≤ 10:0.1, and also useful, approximately *r =* 10:1.

When applied on an implantable medical device, including a stent for blood vessel implantation, typical dosage of a therapeutic compound is 0.01 µg/mm to 100 µg/mm. Typically, a practical maximum is dictated by the polymers, the drug, and the methods of making the device. While other dosages can vary due to the nature of the therapeutic compounds, when zotarolimus or paclitaxel are applied to a stent, typical dosages are 0.01 µg/mm to 20 µg/mm, 0.1 µg/mm to 15 µg/mm, and also useful, 1 µg/mm to 10 µg/mm. However, any dosing regime can be used as long as the ratio of zotarolimus:paclitaxel is kept within approximately 10:7 ≤ *r* ≤ 10:0.01, approximately 10:7 ≤ *r* ≤ 10:0.1, and also useful *r*=10:1 and biological safety is not significantly compromised. Examples of useful stents using zotarolimus and paclitaxel at ratios including 10:7 (zotarolimus:paclitaxel) stent having 5 µg/mm of zotarolimus and 3.5 µg/mm of paclitaxel; for example, in a 10:1 stent, 10 µg/mm of zotarolimus can be applied, and 1 µg/mm of paclitaxel.

Generally speaking, drugs useful in combinations do not adversely affect the desired activity of the other drug in the combination. Thus, one drug in the proposed combination does not inhibit the desired activity, *e*.*g*., anti-proliferative activity, of the other drug. Nor does either drug cause or enhance the degradation of the other drug. However, a drug that might otherwise appear to be unsuitable because, for example, it degrades during sterilization, can in fact be useful because of an interaction with another drug.

### Methods of treatment

The compounds of the invention, including but not limited to those specified in the examples, possess immunomodulatory activity in mammals (especially humans). As immunosuppressants, zotarolimus and derivatives are useful for the treatment and prevention of immune-mediated diseases including the resistance by transplantation of organs or tissue including heart, kidney, liver, medulla ossium, skin, cornea, lung, pancreas, intestinum tenue, limb, muscle, nerves, duodenum, small-bowel, and pancreatic-islet-cell; graft-*versus*-host diseases brought about by medulla ossium transplantation;
autoimmune diseases including rheumatoid arthritis, systemic lupus erythematosus, Hashimoto's thyroiditis, multiple sclerosis, myasthenia gravis, type I diabetes, uveitis, allergic encephalomyelitis, and glomerulonephritis. Further uses include the treatment and prophylaxis of inflammatory and hyperproliferative skin diseases and cutaneous manifestations of immunologically-mediated illnesses, including psoriasis, atopic dermatitis, contact dermatitis and further eczematous dermatitises, seborrhoeis dermatitis, lichen planus, pemphigus, bullous pemphigoid, epidermolysis bullosa, urticaria, angioedemas, vasculitides, erythemas, cutaneous eosinophilias, lupus erythematosus, acne and alopecia areata; various eye diseases (autoimmune and otherwise) including keratoconjunctivitis, vernal conjunctivitis, uveitis associated with Behcet's disease, keratitis, herpetic keratitis, conical cornea, dystrophia epithelialis corneae, corneal leukoma, and ocular pemphigus. In addition reversible obstructive airway disease, which includes conditions including asthma (for example, bronchial asthma, allergic asthma, intrinsic asthma, extrinsic asthma and dust asthma), particularly chronic or inveterate asthma (for example, late asthma and airway hyper-responsiveness), bronchitis, and allergic rhinitis are targeted by compounds of this invention. Inflammation of mucosa and blood vessels including gastric ulcers, vascular damage caused by ischemic diseases and thrombosis. Moreover, hyperproliferative vascular diseases including intimal smooth muscle cell hyperplasia, restenosis and vascular occlusion, particularly following biologically- or mechanically- mediated vascular injury, could be treated or prevented by the compounds of the invention.

Other treatable conditions include but are not limited to ischemic bowel diseases, inflammatory bowel diseases, necrotizing enterocolitis, intestinal inflammations/allergies including Coeliac diseases, proctitis, eosinophilic gastroenteritis, mastocytosis, Crohn's disease and ulcerative colitis; nervous diseases including multiple myositis, Guillain-Barre syndrome, Meniere's disease, polyneuritis, multiple neuritis, mononeuritis and radiculopathy; endocrine diseases including hyperthyroidism and Basedow's disease; hematic diseases including pure red cell aplasia, aplastic anemia, hypoplastic anemia, idiopathic thrombocytopenic purpura, autoimmune hemolytic anemia, agranulocytosis, pernicious anemia, megaloblastic anemia and anerythroplasia; bone diseases including osteoporosis; respiratory diseases including sarcoidosis, fibroid lung and idiopathic interstitial pneumonia; skin disease including dermatomyositis, leukoderma vulgaris, ichthyosis vulgaris, photoallergic sensitivity and cutaneous T cell lymphoma; circulatory diseases including arteriosclerosis, atherosclerosis, aortitis syndrome, polyarteritis nodosa and myocardosis; collagen diseases including scleroderma, Wegener's granuloma and Sjogren's syndrome; adiposis; eosinophilic fasciitis; periodontal disease including lesions of gingiva, periodontium, alveolar bone and substantia ossea dentis; nephrotic syndrome including glomerulonephritis; male pattern alopecia or alopecia senilis by preventing epilation or providing hair germination and/or promoting hair generation and hair growth; muscular dystrophy; Pyoderma and Sezary's syndrome; Addison's disease; active oxygen-mediated diseases, as for example organ injury including ischemia-reperfusion injury of organs (including heart, liver, kidney and digestive tract) which occurs upon preservation, transplantation or ischemic disease (for example, thrombosis and cardiac infarction); intestinal diseases including endotoxin-shock, pseudomembranous colitis and colitis caused by drug or radiation; renal diseases including ischemic acute renal insufficiency and chronic renal insufficiency; pulmonary diseases including toxinosis caused by lung-oxygen or drug (for example, paracort and bleomycins), lung cancer and pulmonary emphysema; ocular diseases including cataracta, siderosis, retinitis, pigmentosa, senile macular degeneration, vitreal scarring and corneal alkali burn; dermatitis including erythema multiforme, linear IgA ballous dermatitis and cement dermatitis; and others including gingivitis, periodontitis, sepsis, pancreatitis, diseases caused by environmental pollution (for example, air pollution), aging, carcinogenesis, metastasis of carcinoma and hypobaropathy; diseases caused by histamine or leukotriene-C₄ release; Behcet's disease including intestinal-, vasculo- or neuro-Behcet's disease, and also Behcet's which affects the oral cavity, skin, eye, vulva, articulation, epididymis, lung, kidney and so on. Furthermore, the compounds of the invention are useful for the treatment and prevention of hepatic disease including immunogenic diseases (for example, chronic autoimmune liver diseases including autoimmune hepatitis, primary biliary cirrhosis and sclerosing cholangitis), partial liver resection, acute liver necrosis (*e*.*g*., necrosis caused by toxin, viral hepatitis, shock or anoxia), B-virus hepatitis, non-A/non-B hepatitis, cirrhosis (including alcoholic cirrhosis) and hepatic failure including fulminant hepatic failure, late-onset hepatic failure and "acute-on-chronic" liver failure (acute liver failure on chronic liver diseases), and moreover are useful for various diseases because of their useful activity including augmention of chemotherapeutic effect, cytomegalovirus infection, particularly HCMV infection, anti-inflammatory activity, sclerosing and fibrotic diseases including nephrosis, scleroderma, pulmonary fibrosis, arteriosclerosis, congestive heart failure, ventricular hypertrophy, post-surgical adhesions and scarring, stroke, myocardial infarction and injury associated with ischemia and reperfusion.

Additionally, compounds of the invention possess FK-506 antagonistic properties. These compounds can thus be used in the treatment of immunodepression or a disorder involving immunodepression. Examples of disorders involving immunodepression include AIDS, cancer, fungal infections, senile dementia, trauma (including wound healing, surgery and shock) chronic bacterial infection, and certain central nervous system disorders. The immunodepression to be treated can be caused by an overdose of an immunosuppressive macrocyclic compound, for example derivatives of 12-(2-cyclohexyl-1-methylvinyl)-13, 19,21,27-tetramethyl-11,28-dioxa-4-azatricyclo[22.3.1.0] octacos-18-ene including FK-506 or rapamycin. The overdosing of such medicaments by patients is quite common upon their realizing that they have forgotten to take their medication at the prescribed time and can lead to serious side effects.

The ability of the compounds of the invention to treat proliferative diseases can be demonstrated according to previously described methods (Bunchman and Brookshire, 1991; Shichiri *et al.,* 1991; Yamagishi *et al.,* 1993). Proliferative diseases include smooth muscle proliferation, systemic sclerosis, cirrhosis of the liver, adult respiratory distress syndrome, idiopathic cardiomyopathy, lupus erythematosus, diabetic retinopathy or other retinopathies, psoriasis, scleroderma, prostatic hyperplasia, cardiac hyperplasia, restenosis following arterial injury or other pathologic stenosis of blood vessels. In addition, these compounds antagonize cellular responses to several growth factors, and therefore possess antiangiogenic properties, making them useful agents to control or reverse the growth of certain tumors, as well as fibrotic diseases of the lung, liver, and kidney.

Aqueous liquid compositions are particularly useful for the treatment and prevention of various diseases of the eye including autoimmune diseases (including, for example, conical cornea, keratitis, dysophia epithelialis corneae, leukoma, Mooren's ulcer, sclevitis and Graves' ophthalmopathy) and rejection of corneal transplantation.

When used in the above or other treatments, a therapeutically effective amount of zotarolimus, for example, can be employed in pure form or, where such forms exist, in pharmaceutically acceptable salt, ester or prodrug form. Alternatively, the compound can be administered as a pharmaceutical composition including the compound of interest in combination with one or more pharmaceutically acceptable excipients. The phrase "therapeutically effective amount" of the compound of the invention means a sufficient amount of the compound to treat disorders, at a reasonable benefit/risk ratio applicable to any medical treatment. It will be understood, however, that the total daily usage of compounds and compositions are decided by an attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of compounds used in this invention administered to a human or lower animal can range from about 0.01 to about 10 mg/kg/day. For purposes of oral administration, doses can be in the range of from about 0.001 to about 3 mg/kg/day. For the purposes of local delivery from a stent, the daily dose that a patient receives depends on the length of the stent. For example, a 15 mm coronary stent can include a drug in an amount ranging from about 1 to about 1,000 micrograms and can deliver that drug over a time period ranging from several hours to several weeks. If desired, the effective daily dose can be divided into multiple doses for purposes of administration; consequently, single dose compositions can include such amounts or submultiples thereof to make up the daily dose. Topical administration can involve doses ranging from 0.001 to 3 mg/kg/day, depending on the site of application.

### Assays

### Testing for neointimal hyperplasia, inflammation and endothelialization after stent implantation

This test can be used to test safety and efficacy. The test exploits the art-accepted porcine coronary overstretch model (Schwartz, 1992) and is usually conducted for approximately 3 to 365 days. Alternatively, rabbits can be used, or any other art-accepted model. Typically, experimental design includes at least a stent control that resembles the experimental stent in every way except for the change of a single variable, including a therapeutic compound or polymer.

In one example, two major coronary arteries are implanted with one test stent each, and the third major coronary artery is implanted with a control stent in each pig. Additional controls can be pigs implanted with three control stents, one each in a major coronary artery. Stents should be the same dimensions, or as close as possible.

Stents are implanted using standard techniques. At the conclusion of the study, animals are euthanized, and the hearts are removed, washed and fixed using standard histological preservation techniques (including formalin, formaldehyde, *etc.*). Stented vessels are excised, then infiltrated and embedded in a suitable medium for sectioning, including methylmethacrylate (MMA), paraffin, or cryomedia. All blocks containing stented vessels are sectioned so that informative sections are obtained; for example, three, in-stent sections and two control sections. Serial thin sections (approximately 5 µm) are usually taken at each level and stained to visualize the cells and tissues (*e.g.,* hematoxylin and eosin (HE) and Masson's Verhoett Elastin (MVE)). Sections are evaluated and scored using an image analysis system or other art accepted methods of morphological data collection and quantification. The data are scored for neointimal area, neointimal thickness, and percent-area stenosis.

Inflammation can also be graded from histological sections as 0, none; 1, scattered inflammatory cells; 2, inflammatory cells encompassing 50% of a strut in at least 25% to 50% of the circumference of the artery; 3, inflammatory cells surrounding a strut in at least 25% to 50% of the circumference of the artery (*see* (Carter *et al.,* 2004) for more details).

### Elution of drug from stents

For assessment of *in vitro* drug elution, medical devices can be placed in an appropriate solution to dissolve any drug on the medical device, including 10 mM acetate buffer (pH = 4.0) with 1% Solutol HS 15 warmed to 37° C in the case of zotarolimus. A solubilizing agent may be needed if the drugs have very low water solubility. The dissolution medium can buffered to minimize the degradation of drugs that occurs at pH's above 6. Buffering at pH 4 solves this problem in the case of olimus drugs. If the eluted drugs have minimum dissociation at these pH ranges, pH should have little impact on elution rate. Samples are pulled from the dissolution bath at selected time intervals using, for example, a syringe sampler fitted with only Teflon, stainless steel or glass surfaces. In those cases where a time course study is desired, aliquots can be periodically collected, including after 15 min, 30 min, 1 hr, 2 hr, 4 hr, 6 hr, 8 hr, 12 hr and 24 hr. The samples are assayed for zotarolimus concentration *via* HPLC. Data are expressed as drug-eluted in micrograms and mean-percent eluted.

In the HPLC method, it is sometimes necessary to use column-switching to minimize Solutol contamination of the analytical column and to allow rinsing of the guard column; otherwise, the system becomes coated with the Solutol and the chromatographic retention changes dramatically. The samples are first injected onto a guard column. Once the analyte peak elutes from the guard column and passes onto the analytical column, the guard column is switched out of the analytical path. The guard column was then washed to remove the Solutol prior to the next injection.

### Drug penetration assay

This assay can be used to demonstrate the ability of the device to effectively deliver the therapeutic compound to the tissues adjacent to the site of implantation. Ideally, a better system delivers the loaded therapeutic compound(s) to tissues adjacent to it, and not in the blood (*i.e*., systemically).

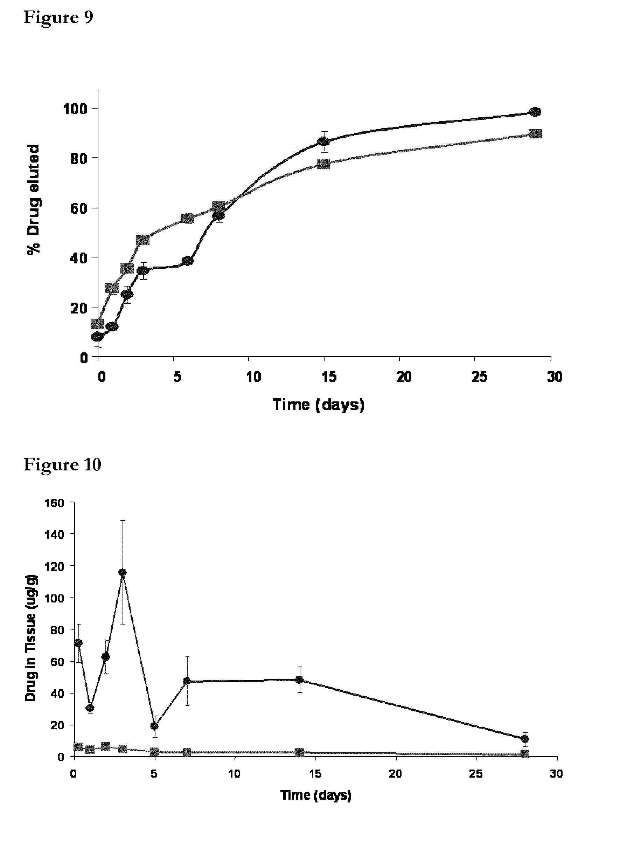

At pre-chosen time points (one example of where to start is found in **Figure 10**) stents that had been implanted as indicated in *Testing for neointimal hyperplasia, inflammation and endothelialization after stent implantation* are removed. The arterial tissue is removed from the stents, and the amount of drug that had penetrated the arterial walls adjacent to the stent is assayed for the presence and concentration of the target therapeutic compound(s). The data are then typically averaged, and plotted in a graph where the *x*-axis represent time, and the *y*-axis represent the amount of drug in tissue. Any art-accepted model for determining the concentration of a therapeutic compound can be used, including HPLC or other chromatography, immunoassays, activity assays, or other method of identification.

### Blood concentration assay

This assay can be used to demonstrate the relative efficacy of a therapeutic compound delivered from the system of the invention to not enter the blood stream and is ideally used in conjunction with *Drug penetration assay.* Ideally, a better system delivers the loaded therapeutic compound(s) to tissues adjacent to it, and not in the blood (*i.e.,* systemically).

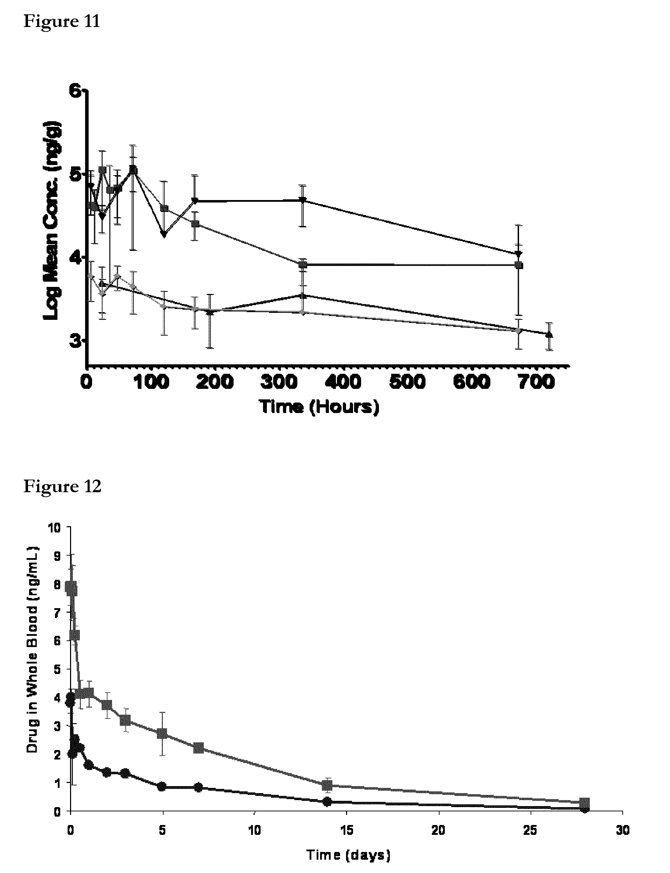

At pre-chosen time points (one example of where to start is found in **Figure 11**) blood samples from the subjects that have stents that had been implanted as indicated in *Testing for neointimal hyperplasia, inflammation and endothelialization after stent implantation* are collected by any art-accepted method, including venipuncture. Blood concentrations of the loaded therapeutic compounds are determined using any art-accepted method of detection, including immunoassay, chromatography (including liquid/liquid extraction HPLC tandem mass spectrometric method (LC-MS/MS) (Ji *et al.,* 2004)), and activity assays.

### Kits

The systems of the invention can be included in a kit, container, pack, or dispenser together with instructions for administration and use. When supplied as a kit, the different components of the composition can be packaged in separate containers. Kits can also include reagents in separate containers.

### Containers or vessels

The reagents included in the kits can be supplied in containers or packaging of any sort such that the life of the different components are preserved and are not adsorbed or altered by the materials of the container. For example, sealed glass ampules may contain lyophilized buffer that has been packaged under a neutral non-reacting gas, such as nitrogen. Ampules may consist of any suitable material, such as glass, organic polymers, such as polycarbonate, polystyrene, etc., ceramic, metal or any other material typically employed to hold reagents. Other examples of suitable containers include bottles that can be fabricated from similar substances as ampules, and envelopes, that consist of foil-lined interiors, such as aluminum or an alloy. Examples of containers include test tubes, vials, flasks, bottles and syringes. Containers can have a sterile access port, such as a bottle having a stopper that can be pierced by a hypodermic injection needle. Other containers can have two compartments that are separated by a readily removable membrane that upon removal permits the components to mix. Removable membranes can be glass, plastic, rubber, etc.

### Instructional materials

Kits can also be supplied with instructional materials. Instructions can be printed on paper or other substrate, and/or can be supplied as an electronic-readable medium, such as a floppy disc, CD-ROM, DVD-ROM, Zip disc, videotape, audiotape, mini-disc, cassette tape or provided by calling a prescribed telephone number. Detailed instructions may not be physically associated with the kit; instead, a user may be directed to an Internet web site specified by the manufacturer or distributor of the kit, or supplied as electronic mail.

The following examples are provided to help illustrate the invention, not limit it. Table "Examples" briefly summarizes the contents of this section.

**TABLE**

| Examples | |
|---|---|
| Example | Contents |
| 1 | Synthesis of zotarolimus (less polar isomer) |
| 2 | Synthesis of zotarolimus (more polar isomer) |
| 3 | Zotarolimus effects on neointimal formation |
| 4 | Rate of release of zotarolimus drug from 316L electropolished stainless steel coupons coated with a biocompatible polymer having phosphorylcholine side groups |
| 5 | Loading and release of zotarolimus from 15 mm BiodivYsio drug delivery stents |
| 6 | *In vivo* zotarolimus dose experiments |
| 7 | Elution experiments of beneficial agents |
| 8 | Neointimal formation *in vivo* after stent implantation |
| 9 | Comparison of the elution profiles, tissue concentration and blood concentration of zotarolimus-polymer coated stents (ZoMaxxTM) and rapamycin-coated stents (Cypher®) implanted in rabbit iliac arteries |
| 10 | Extended studies of zotarolimus-coated and rapamycin-coated stents *in vivo* |
| 11 | 28 day overlapping drug-eluting stent study |
| 12 | Clinical example |
| 13 | Clinical example (prophetic) |

### Example 1 Synthesis of 42-Epi-(tetrazolyl)-rapamycin (less polar isomer)

### Example 1A

A solution of rapamycin (100 mg, 0.11 mmol) in dichloromethane (0.6 ml) at -78° C under a nitrogen atmosphere was treated sequentially with 2,6-lutidine (53 µl, 0.46 mmol, 4.3 eq.) and trifluoromethanesulfonic anhydride (37 µl, 0.22 mmol), and stirred thereafter for 15 minutes, warmed to room temperature and eluted through a pad of silica gel (6 ml) with diethyl ether. Fractions containing the triflate were pooled and concentrated to provide the designated compound as an amber foam.

### Example 1B 42-Epi-(tetrazolyl)-rapamycin (less polar isomer)

A solution of Example 1A in isopropyl acetate (0.3 ml) was treated sequentially with diisopropylethylamine (87 ml, 0.5 mmol) and 1H-tetrazole (35 mg, 0.5 mmol), and thereafter stirred for 18 hours. This mixture was partitioned between water (10 ml) and ether (10 ml). The organics were washed with brine (10 ml) and dried (Na₂SO₄). Concentration of the organics provided a sticky yellow solid which was purified by chromatography on silica gel (3.5 g, 70-230 mesh) eluting with hexane (10 ml), hexane:ether (4:1(10 ml), 3:1(10 ml), 2:1(10 ml), 1:1 (10 ml)), ether (30 ml), hexane:acetone (1:1 (30ml)). One of the isomers was collected in the ether fractions.
MS (ESI) m/e 966 (M)⁻

### Example 2 42-Epi-(tetrazolyl)-rapamycin (more polar isomer)

### Example 2A 42-Epi-(tetrazolyl)-rapamycin (more polar isomer)

Collection of the slower moving band from the chromatography column using the hexane:acetone (1:1) mobile phase in Example 1B provided the designated compound, which is zotarolimus.
MS (ESI) m/e 966 (M)⁻.

### In vitro Assay of Biological Activity

The immunosuppressant activity of zotarolimus was compared to rapamycin and two rapamycin analogs: 40-epi-N-[2'-pyridone]-rapamycin and 40-epi-N-[4'-pyridone]-rapamycin. The activity was determined using the human mixed lymphocyte reaction (MLR) assay described (Kino *et al.,* 1987). The results of the assay demonstrate that the compounds of the invention are effective immunomodulators at nanomolar concentrations, as shown in Table 1.

**TABLE 1**

| Example | Human MLR IC₅₀ ± S.E.M.(nM) |
|---|---|
| Rapamycin | 0.91 ± 0.36 |
| 2-pyridone | 12.39 ± 5.3 |
| 4-pyridone | 0.43 ± 0.20 |
| Example 1 | 1.70 ± 0.48 |
| Example 2 (zotarolimus) | 0.66 ± 0.19 |

The pharmacokinetic behaviors of Example 1 and Example 2 were characterized following a single 2.5 mg/kg intravenous dose in cynomolgus monkey (n=3 per group). Each compound was prepared as 2.5 mg/ml solution in a 20% ethanol:30% propylene glycol:2% cremophor EL:48% dextrose 5% in water vehicle. The 1 ml/kg intravenous dose was administered as a slow bolus (~1-2 minutes) in a saphenous vein of the monkeys. Blood samples were obtained from a femoral artery or vein of each animal prior to dosing and 0.1 (IV only), 0.25, 0.5, 1, 1.5, 2, 4, 6, 9, 12, 24, and 30 hours after dosing. The EDTA preserved samples were thoroughly mixed and extracted for subsequent analysis.

An aliquot of blood (1.0 ml) was hemolyzed with 20% methanol in water (0.5 ml) containing an internal standard. The hemolyzed samples were extracted with a mixture of ethyl acetate and hexane (1:1 (v/v), 6.0 ml). The organic layer was evaporated to dryness with a stream of nitrogen at room temperature. Samples were reconstituted in methanol: water (1:1, 150 µl). The title compounds (50 µl injection) were separated from contaminants using reverse phase HPLC with UV detection. Samples were kept cool (4° C) through the run. All samples from each study were analyzed as a single batch on the HPLC.

Area under the curve (AUC) measurements of Example 1, Example 2 and the internal standard were determined using the Sciex MacQuan™ software. Calibration curves were derived from peak area ratio (parent dr µg/internal standard) of the spiked blood standards using least squares linear regression of the ratio *versus* the theoretical concentration. The methods were linear for both compounds over the range of the standard curve (correlation > 0.99) with an estimated quantitation limit of 0.1 ng/ml. The maximum blood concentration (Cₘₐₓ) and the time to reach the maximum blood concentration (Tₘₐₓ) were read directly from the observed blood concentration-time data. The blood concentration data were submitted to multi-exponential curve fitting using CSTRIP to obtain estimates of pharmacokinetic parameters. The estimated parameters were further defined using NONLIN84. The area under the blood concentration-time curve from 0 to t hours (last measurable blood concentration time point) after dosing (AUC₀₋ₜ) was calculated using the linear trapezoidal rule for the blood-time profiles. The residual area extrapolated to infinity, determined as the final measured blood concentration (Cₜ) divided by the terminal elimination rate constant (β), and added to AUC₀₋ₜ to produce the total area under the curve (AUC₀₋ₜ).

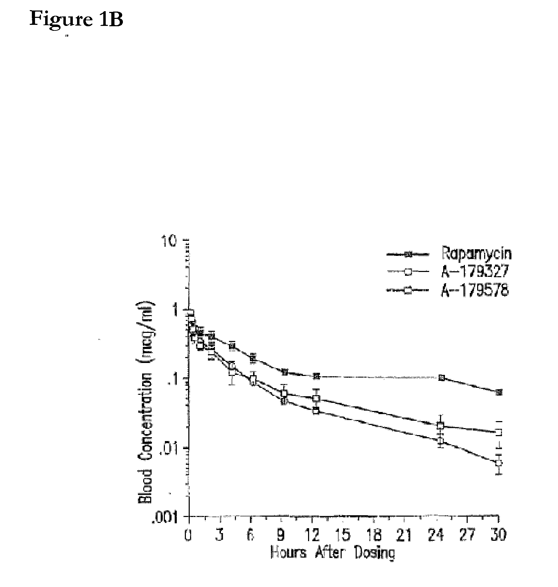

As shown in **Figure 1B** and Table 2, both Example 1 and Example 2 had a surprisingly substantially shorter terminal elimination half-life (t_{1/2}) when compared to rapamycin. Thus, only the compounds of the invention provide both sufficient efficacy (Table 1) and a shorter terminal half-life (Table 2).

**TABLE 2**

| Compound | AUC (ng·hr/ml) | t_{1/2} (hours) |
|---|---|---|
| Rapamycin | 6.87 | 16.7 |
| 2-pyridone | 2.55 | 2.8 |
| 4-pyridone | 5.59 | 13.3 |
| Example 1 | 2.35 | 5.0 |
| Example 2 (zotarolimus) | 2.38 | 6.9 |

### Example 3 Zotarolimus effects on neointimal formation

The purpose of this example was to determine the effects of a rapamycin analog on neointimal formation in porcine coronary arteries in which stents were placed. This example illustrates that the rapamycin analog zotarolimus, when compounded and delivered from the Biocompatibles BiodiviYsio PC Coronary stent favorably affects neointimal hyperplasia and lumen size in porcine coronary arteries. This finding suggests that a combination from a drug-eluting stent including zotarolimus can be of substantial clinical benefit if properly applied in humans by limiting neointimal hyperplasia.

This study exploited a porcine coronary stent model since domestic swine yield results that are comparable to other investigations assaying neointimal hyperplasia in human subjects.

The example tested zotarolimus eluted from coronary stents placed in juvenile farm pigs, and compared these results with control stents. The control stents had polymer alone on the struts. This is important, for the polymer itself must not stimulate neointimal hyperplasia to a substantial degree. As the eluted drug disappears, an inflammatory response to the polymer could conceivably result in a late "catch-up phenomenon" where the restenosis process is not stopped, but instead slowed. This phenomenon would result in restenosis at late dates in human subjects.

Stents were implanted in two blood vessels in each pig. Pigs used in this model were generally 2-4 months old and weighed 30-40 kg. Two coronary stents were thus implanted in each pig by visually assessing a normal stent:artery ratio of 1.1-1.2.

Beginning on the day of the procedure, pigs were given oral aspirin (325 mg daily) and continued for the remainder of their course. General anesthesia was achieved by means of intramuscular injection followed by intravenous ketamine (30 mg/kg) and xylazine (3 mg/kg). Additional medication at the time of induction included atropine (1 mg) and flocillin (1 g) administered intramuscularly. During the stenting procedure, an intra-arterial bolus of 10,000 units of heparin was administered.

Arterial access was obtained by cutdown on the right external carotid and placement of an 8F sheath. After the procedure, the animals were maintained on a normal diet without cholesterol or other special supplementation.

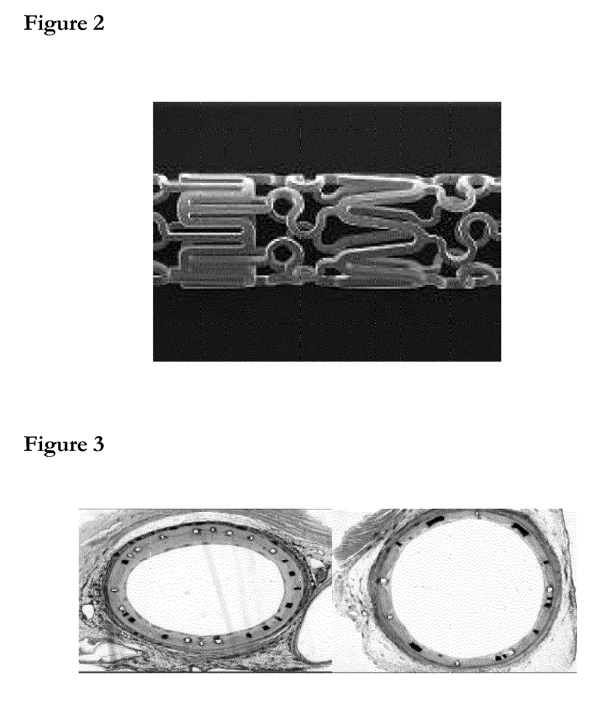

The BiodivYsio stent was used with nominal vessel target size of 3.0 mm. See **Figure 2****.** Two coronary arteries per pig were assigned at random to deployment of the stents. The stent was either a drug eluting stent (polymer plus drug stent) or a stent coated with a polymer only (polymer only stent). The stents were delivered by means of standard guide catheters and wires. The stent balloons were inflated to appropriate sizes for less than 30 seconds.

Each pig had one polymer only stent and one polymer plus drug stent placed in separate coronary arteries, so that each pig would have one stent for drug and one for control.

A sample size of 20 pigs total was chosen to detect a projected difference in neointimal thickness of 0.2 mm with a standard deviation of 0.15 mm, at a power of 0.95 and beta 0.02.

Animals were euthanized at 28 days for histopathologic examination and quantification. Following removal of the heart from the perfusion pump system, the left atrial appendage was removed for access to the proximal coronary arteries. Coronary arterial segments with injuries were dissected free of the epicardium. Segments containing lesions was isolated, thereby allowing sufficient tissue to contain uninvolved blood vessel at either end. The foregoing segments, each roughly 2.5 cm in length, were embedded and processed by means of standard plastic embedding techniques. The tissues were subsequently processed and stained with hematoxylin-eosin and elastic-van Gieson techniques.

Low and high power light microscopy were used to make length measurements in the plane of microscopic view by means of a calibrated reticle and a digital microscopy system connected to a computer employing calibrated analysis software.

The severity of vessel injury and the neointimal response were measured by calibrated digital microscopy. The importance of the integrity of the internal elastic lamina is well-known to those skilled in the art. A histopathologic injury score in stented blood vessels has been validated as being closely related to neointimal thickness. This score is related to depth of injury and as shown in Table 3:

**TABLE 3**

| Histopathologic injury scoring | |
|---|---|
| Score | Description of Injury |
| 0 | Internal elastic lamina intact; endothelium typically denuded, media compressed but not lacerated. |
| 1 | Internal elastic lamina lacerated; media typically compressed but not lacerated. |
| 2 | Internal elastic lacerated; media visibly lacerated; external elastic lamina intact but compressed. |
| 3 | External elastic lamina lacerated; typically large lacerations of media extending through the external elastic lamina; coil wires sometimes residing in adventitia. |

This quantitative measurement of injury was assessed for all stent wires of each stent section. The calibrated digital image was also used to measure at each stent wire site the neointimal thickness. Lumen area, area contained with the internal elastic lamina, and area within the external elastic lamina were also measured.

At each stent wire site for a given section, the neointimal thickness was averaged to obtain a mean injury score for each section. The measurement of neointimal thickness was made to the abluminal side of the stent wire, because the neointimal in all cases includes this thickness.

The mid-stent segment was used for measurement, analysis, and comparison. Data were also recorded (and included in the data section of this report) for proximal and distal segments.

The data analysis methods for this study did not need to take into account variable arterial injury across treatment/control groups, because mild to moderate injury is sensitive enough to detect treatment differences. Paired t-testing was performed to compare variables across the polymer only stents (control group) and polymer plus drug stents (treatment group). No animal died in this study before scheduled time points

Table 4 shows the summary results for all data for mean injury and neointimal thickness for each stent, including proximal, mid, and distal segments. Table 4 also shows lumen size, percent stenosis, and artery size as measured by the internal elastic laminae (IEL) and external elastic laminae (EEL).

**TABLE 4**

| Summary: All Measures (Distal, Mid. Proximal) | | | | | | | | | |
|---|---|---|---|---|---|---|---|---|---|
| ID | prox ref | dist ref | lumen | IEL | EEL | mean injury | % stenosis | Neointimal area | NIT |
| Control | Distal | | | | | | | | |
| Mean | 4.46 | 3.96 | 4.88 | 7.66 | 9.00 | 0.22 | 36.10 | 2.79 | 0.41 |
| SD | 1.20 | 1.16 | 1.30 | 1.15 | 1.10 | 0.26 | 15.41 | 1.29 | 0.17 |
| | | | | | | | | | |
| Control | Mid | | | | | | | | |
| Mean | 4.46 | 3.96 | 4.94 | 7.71 | 9.08 | 0.08 | 36.23 | 2.77 | 0.38 |
| SD | 1.20 | 1.16 | 1.44 | 1.07 | 1.15 | 0.14 | 14.93 | 1.20 | 0.16 |
| | | | | | | | | | |
| Control | Proximal | | | | | | | | |
| Mean | 4.46 | 3.96 | 5.11 | 7.89 | 9.30 | 0.15 | 35.35 | 2.78 | 0.38 |
| SD | 1.20 | 1.16 | 1.38 | 1.33 | 1.42 | 0.22 | 11.94 | 1.04 | 0.12 |
| | | | | | | | | | |
| Test | Distal | | | | | | | | |
| Mean | 4.26 | 3.41 | 6.04 | 7.70 | 9.01 | 0.26 | 22.35 | 1.66 | 0.25 |
| SD | 1.26 | 0.96 | 1.55 | 1.49 | 1.47 | 0.43 | 8.58 | 0.58 | 0.06 |
| Test | Mid | | | | | | | | |
| Mean | 4.26 | 3.41 | 6.35 | 7.75 | 8.98 | 0.04 | 18.71 | 1.41 | 0.22 |
| SD | 1.26 | 0.96 | 1.29 | 1.18 | 1.31 | 0.07 | 5.68 | 0.33 | 0.05 |
| | | | | | | | | | |
| Test | Proximal | | | | | | | | |
| Mean | 2.56 | 2.15 | 3.31 | 4.06 | 4.66 | 0.19 | 16.79 | 1.29 | 0.18 |
| SD | 1.66 | 1.37 | 2.39 | 3.48 | 4.15 | 0.13 | 9.97 | 0.80 | 0.12 |

There was no statistically significant difference for neointimal area or thickness across proximal, mid, or distal segments within the test group (polymer plus drug stents) or control groups (polymer only stents). This observation is quite consistent with prior studies, and thus allows use of only the mid segment for statistical comparison of test devices (polymer plus drug stents) vs. control devices (polymer only stents).

Table 5 shows the statistical t-test comparisons across test groups and control groups. There was a statistically significant difference in neointimal thickness, neointimal area, lumen size, and percent lumen stenosis, the drug eluting stent being clearly favored. Conversely, there were no statistically significant differences between the test group (polymer plus drug stents) and the control group (polymer only stents) for mean injury score, external elastic laminae, or internal elastic laminae areas.

**TABLE 5**

| Statistical Comparison of Test *vs*. Control Parameters: Mid-Section Data (t-test Statistics) | | | | | | | |
|---|---|---|---|---|---|---|---|
| Parameter | Difference | t-test | DF | Std Error | Lower 95% | Upper 95% | p |
| Lumen | -1.17 | -2.28 38 | | 0.52 | -2.21 | -0.13 | 0.029 |
| IEL | 0.03 | 0.088 | 38 | 0.36 | -0.71 | 0.78 | 0.93 |
| EEL | 0.2 | 0.499 | 38 | 0.39 | -0.599 | 0.99 | 0.62 |
| NI Thickness | 0.18 | 5.153 | 38 | 0.034 | 0.106 | 0.244 | <.0001 |
| NI Area | 1.21 | 3.62 | 38 | 0.33 | 0.53 | 1.88 | 0.0008 |
| Mean Injury | 0.038 | 1.137 | 38 | 0.033 | -0.02 | 0.106 | 0.26 |
| % Stenosis | 14.54 | 2.97 | 38 | 4.9 | 4.61 | 24.47 | 0.005 |

The reference arteries proximal and distal to the stented segments were observed, and quantitated. These vessels appeared normal in all cases, uninjured in both the control group (polymer only stents) and the test group (polymer plus drug stents). See **Figures 3A and 3B.** The data in Table 5, below, show there were no statistically significant differences in size between the stents in the control group and the stents in the test group.

**TABLE 5**

| Stent size | | |
|---|---|---|
| | Proximal Reference Diameter (mm) | Distal Reference Diameter (mm) |
| Control (mean ± SD) | 4.46 + 1.20 | 3.96 ± 1.16 |
| Test (mean ± SD) | 4.26 ± 1.26 | 3.41 ± 0.96 |

The data suggest that statistically significant differences exist, and these differences favor the stent that elutes zotarolimus. The stent of this invention results in lower neointimal area, lower neointimal thickness, and greater lumen area. There were no significant differences within the test group (polymer plus drug stents) and the control group (polymer only stents) for neointimal or injury parameters. There were no significant differences in artery sizes (including the stent) for the control group compared to the test group. These latter findings suggest no significant difference in the arterial remodeling characteristics of the polymeric coating including the drug.

At most, mild inflammation was found on both the polymer plus drug stent and the polymer only stent. This finding suggests that the polymer exhibits satisfactory biocompatibility, even without drug loading. Other studies show that when drug has completely gone from the polymer, the polymer itself creates enough inflammation to cause neointima. This phenomenon can be responsible for the late catch-up phenomenon of clinical late restenosis. Because the polymer in this example did not cause inflammation in the coronary arteries, late problems related to the polymer after the drug is exhausted are unlikely.

In conclusion, a stent that includes zotarolimus with a polymer showed a reduction in neointimal hyperplasia in the porcine model when placed in a coronary artery.

### Example 4 Rate of release ot zotarolimus drug from 316L electropolished stainless steel coupons coated with a biocompatible polymer having hosphorylcholine side groups

The purpose of this example was to determine the rate of release of zotarolimus drug from 316L electropolished stainless steel coupons coated with a biocompatible polymer having phosphorylcholine side groups.

Rubber septa from lids from HPLC vials were removed from the vials and placed into glass vials so that the "Teflon" side faced up. These septa served as supports for the test samples. The test samples were 316L stainless steel coupons that had been previously coated with a biocompatible polymer having phosphorylcholine side groups (PC polymer). Coronary stents are commonly made of 316L stainless steel and can be coated with the PC polymer to provide a depot site for loading drugs. The coated coupons, which serve to simulate stents, were placed onto the septa. By using a glass Hamilton Syringe, a solution of zotarolimus and ethanol (10 µl) was applied to the surface of each coupon. Zotarolimus (30.6 mg) was dissolved in 100% ethanol (3.0 ml). The syringe was cleaned with ethanol between each application. The cap to the glass vial was placed on the vial loosely, thereby assuring proper ventilation. The coupon was allowed to dry for a minimum of 1.5 hours. Twelve (12) coupons were loaded in this way - six being used to determine the average amount of drug loaded onto the device and six being used to measure the time needed to release the drug from the devices.

To determine the total amount of zotarolimus loaded onto a coupon, a coupon was removed from the vial and placed into 50/50 acetonitrile/ 0.01M phosphate buffer (pH 6.0, 5.0 ml). The coupon was placed onto a 5210 Branson sonicator for one hour. The coupon was then removed from the solution, and the solution was assayed by HPLC.

The time release studies were performed by immersing and removing the individual coupons from fresh aliquots (10.0 ml) of 0.01 M phosphate buffer at a pH of 6.0 at each of the following time intervals - 5, 15, 30 and 60 minutes. For the remaining time points of 120, 180, 240, 300, 360 minutes, volumes of 5.0 ml of buffer were used. To facilitate mixing during the drug release phase, the samples were placed onto an Eberbach shaker set at low speed. All solution aliquots were assayed by HPLC after the testing of the last sample was completed.

The HPLC analysis was performed with a Hewlett Packard series 1100 instrument having the following settings:

| | |
|---|---|
| Injection Volume | = 100 µl |
| Acquisition Time | = 40 minutes |
| Flow Rate | = 1.0 ml/min |
| Column Temperature | = 40 °C |
| Wavelength | = 278 nm |
| Mobile Phase | = 65% Acetonitrile/35% H₂O |
| Column | = YMC ODS-A S5 µm,4.6 x 250 mm Part No. A12052546WT |

The results from the above experiment showed the following release data (Table 6):

**TABLE 6**

| Time (min.) | Percent Release | Standard Deviation |
|---|---|---|
| 0.00 | 0.00 | 0.00 |
| 5.00 | 1.87 | 1.12 |
| 15.00 | 2.97 | 1.47 |
| 30.00 | 3.24 | 1.28 |
| 60.00 | 3.29 | 1.29 |
| 120.00 | 3.92 | 1.28 |
| 180.00 | 4.36 | 1.33 |
| 240.00 | 4.37 | 1.35 |
| 300.00 | 6.34 | 2.07 |
| 360.00 | 7.88 | 1.01 |

### Example 5 Loading and release of zotarolimus from 15 mm BiodivYsio drug delivery stents

This examples demonstrates the loading and release of zotarolimus from 15 mm BiodivYsio drug delivery stents.

To load the stents with drug, a solution of zotarolimus in ethanol at a concentration of 50 mg/ml was prepared and dispensed into twelve vials. Twelve individual polymer-coated stents were placed on fixtures designed to hold the stent in a vertical position and the stents were immersed vertically in the drug solution for five minutes. The stents and fixtures were removed from the vials and excess drug solution was blotted away by contacting the stents with an absorbent material. The stents were then allowed to dry in air for 30 minutes in an inverted vertical position.

The stents were removed from the fixtures, and each stent was placed into 50/50 acetonitrile/phosphate buffer (pH 5.1, 2.0 ml) and sonicated for one hour. The stents were removed from the solution and solutions were assayed for concentration of drug, which allowed calculation of the amount of drug originally on the stents. This method was independently shown to remove at least 95% of the drug from the stent coating. On average, the stents contained 60 micrograms of drug ± 20 micrograms.

The drug-loaded stents were placed on the fixtures and placed into 0.01 M phosphate buffer (pH = 6.0, 1.9 ml) in individual vials. These samples were placed onto a Eberbach shaker set at low speed to provide back-and-forth agitation. To avoid approaching drug saturation in the buffer, the stents were transferred periodically to fresh buffer vials at the following points: 15, 30, 45, 60, 120, 135, 150, 165, 180, 240, 390 minutes. The dissolution buffer vials were assayed by HPLC for the drug concentration at the end of the drug release period studied. The data, represented as % cumulative release of the drug as a function of time, is shown in tabular form below (Table 7):

**TABLE 7**

| Time (min) | % Cumulative Release of Drug |
|---|---|
| 15 | 0.3 |
| 30 | 1.1 |
| 45 | 2.1 |
| 60 | 3.2 |
| 120 | 4.3 |
| 135 | 5.9 |
| 150 | 6.3 |
| 165 | 6.8 |
| 180 | 7.4 |
| 240 | 10.8 |
| 390 | 13.2 |

### Example 6 In vivo zotarolimus dose experiments

The purpose of this example was to evaluate the safety of different drug dosages in a pig over-stretch model. Drug was delivered from the BiodivYsio OC stent (15 mm) coated with zotarolimus. In-stent neointima formation was measured at four time intervals - 3 days, 1 month, and 3 months - in the coronary arteries of adult miniature swine. Forty (40) animals were studied at each time interval (10 animals per dose). Each animal received one drug-coated stent and one control stent. The control stent contained no drug. Table 8 shows the dosing scheme for swine efficacy study.

**TABLE 8**

| | Dose group 1 (µg) | Dose group 2 (µg) | Dose group 3 (µg) | Dose group 4 (µg) |
|---|---|---|---|---|
| zotarolimus per stent | 15 | 45 | 150 | 400 |
| zotarolimus per mm of stent | 1 | 3 | 10 | 27 |

Potential local tissue toxicity was assessed at all time intervals by examining histopathologic changes in the stented region, adjacent coronary segments, perivascular tissue, and subserved myocardium. The mortality, angiographic implant and restudy data, histomorphometry data, and stent site histopathology were studied.

### Three-Day Group

Histopathology in combination with scanning electron microscopy provided information regarding the short-term response to the implanted stent. The responses were similar in the control group and all dose groups, and the responses involved compression of the tunica media without remarkable necrosis, an accumulation of thrombus and inflammatory cells mostly localized to the stent struts, and early evidence of endothelial recovery and smooth muscle cell invasion of the thin mural thrombi. There were no extensive thrombi or remarkable intramural hemorrhages. The adventitia in some samples displayed either focal or diffuse inflammatory infiltrates, and occasionally, there was plugging or congestion of the vasa vasora. There was no evidence of medial necrosis in any sample.

Scanning electron microscopy showed similar appearance of the luminal surface three days after the implant of the coronary stent in all dose groups. The shape of the stent was clearly embedded in a thin layer of tissue. The endothelium was intact between the struts and even over the struts; a confluent or nearly confluent layer of endothelial-like cells had covered the luminal surface. There were scattered adherent platelets, platelet microthrombi, and leukocytes over the stents and on the intact remnant endothelium in the inter-strut spaces. In arteries with more severe stent-induced vessel damage, there were more substantial mural thrombi, but the extent of endothelial recovery over the stent struts did not appear retarded, regardless of the dosage of zotarolimus.

### One-Month Group

The histomorphometry data for the one-month series indicated a significant inhibitory effect of locally eluted zotarolimus on neointima formation in stented coronary arteries of swine. Intima area normalized to injury score was significantly decreased for dose groups 3 and 4 (10 and 27 µg/mm) as compared with the control; there were also trends for decreases in absolute intima area and intima thickness for both dose groups 3 and 4 as compared with the control, and a tendency towards decreased histologic % stenosis for dose group 3 as compared with the control.

The control stents displayed morphology typical of stents implanted in coronary arteries of Yucatan miniature swine at one month. The tunica media was compressed or thinned without necrosis subjacent to profiles of stent struts; there were only occasional inflammatory infiltrates; and the neointima ranged in size from relatively thin to moderately thin, and were composed of spindle-shaped and stellate cells in an abundant extracellular matrix, with only rare small foci of fibrinoid material around the profiles of the stent struts. The drug-coated stents showed similar compression of the tunica media without any substantial necrosis at any dose; like control devices, there was little inflammation present. The neointima was notably thinner in dose groups 3 and 4, in some cases being composed of only a few layers of cells. In all dose groups, there were substantial numbers of samples in which moderately sized fibrinoid deposits and inspisated thrombi were observed in the deep neointima. These were usually associated with the stent struts but sometimes extended between strut profiles. However, in no case was there exposure of thrombus on the luminal surface, as the deposits were encapsulated within tibrocellular tissue and covered with a flattened layer of periluminal endothelial-like cells.

Scanning electron microscopy confirmed that a confluent layer of endothelial or endothelial-like cells covered the entire stented surface, and there was no difference between drug-coated stents and control stents in terms of adherence of blood elements; leukocytes were present in approximately equal numbers in all groups. These findings demonstrate that while zotarolimus was associated with decreased neointima formation and persistent mural thrombi, sufficient vessel wall healing in response to stent injury had occurred within one month after the stent had been implanted. This vessel wall healing had rendered the luminal surface non-reactive for platelet adhesion and thrombus formation, and minimally reactive for leukocyte adherence. Additionally, there was no evidence of vessel wall toxicity even at the highest dose (27 µg/mm), as there was no medial necrosis or stent malapposition.

### Three-Month Group

There were no significant differences between the dose groups for any histomorphometric parameters of stented coronary arterial dimension in the three-month period of the study. However, there were weak trends for decreases in the two primary variables describing neointima formation - the cross-sectional area and the % area stenosis of the lumen.

The histopathologic appearance of the control stents in the swine coronary artery samples at three months after the implant appeared similar to that of the controls from the one-month group, and similar to those of all the groups in the three-month period. All samples showed fibrocellular neointima formation with mostly spindle-shaped smooth muscle-like cells in the neointima and a confluent squamous periluminal cell layer. There were no intramural hemorrhages or persistent fibrinoid deposits in the neointima; however some samples, particularly those with thicker neointima, showed evidence of prior thrombus accumulation and subsequent organization in the form of neovascularization in the neointima. On occasion, samples showed evidence of moderate to severe inflammatory reactions localized to the stent struts, associated with destruction of the tunica media architecture. These were most often associated with thicker neointima as well. However, these were few in number and were found in the control group as well as in the drug-coated stent groups. It is presumed that these represented either animal-specific generalized reactions to the implanted stent, evidence of contamination of the stent, or some combination of these two factors, and is commonly found at an incidence of about 10-15% in the studies of stent implants in swine coronary arteries. There was no evidence of necrosis of the tunica media or separation of the media from the stent in any sample. The adventitia of most three-month implants appeared to have somewhat greater neovascularization than did the one-month implants, but this did not appear related to control or test stent group. Scanning electron microscopy demonstrated confluent endothelium with rare adherent blood cells in the control group and all dose groups.

### Conclusions

The stent coated with zotarolimus reduced in-stent neointima formation in swine coronary arteries and provided clear evidence of a biologic drug effect (unresorbed thrombus/fibrin deposits of neointima) at one month. There was a weak tendency for the stent coated with zotarolimus to show a persistent inhibitory effect at the longer-term time interval of three months. There was no local coronary arterial wall toxicity in the form of medial necrosis or stent malapposition associated with any dose group, including the highest dose of approximately 27 µg/mm stent length at any time interval examined. All stents were well incorporated into the tissue, and there was evidence of stable healing responses in the form of tibrocellular neointimal incorporation and endothelial coverage at the one-month interval and at the three-month interval. The trend towards a sustained inhibitory effect at three months after the stent was implanted in this animal is surprising and provides evidence for potentially persistent effects in preventing clinical restenosis resulting from implanted stents.

### Example 7 Elution experiments of beneficial agents

Drug-eluting stents used in this study were similar to the ZoMaxx^{™} Drug-Eluting Stent System (Abbott Vascular; Redwood City, CA) in that they contained 10 µg/mm zotarolimus and a phosphorylcholine-based polymer (PC) topcoat designed to slow the elution of zotarolimus through diffusion. The stents in this study were made of stainless steel and four different groups were constructed with the only difference being that the amount of topcoat was varied by weight (0, 2, 5, or 10 µg/min).

### In Vitro Drug Elution

For assessment of *in vitro* drug elution, stents (n=12 for each group) were expanded and then placed in a solution of 10 mM acetate buffer (pH = 4.0) with 1% Solutol HS 15 heated to 37°C. Aliquots were collected after 15 min, 30 min, 1 hr, 2 hr, 4 hr, 6 hr, 8 hr, 12 hr and 24 hr and assayed for zotarolimus concentration via HPLC. Data are expressed as mean percent eluted.

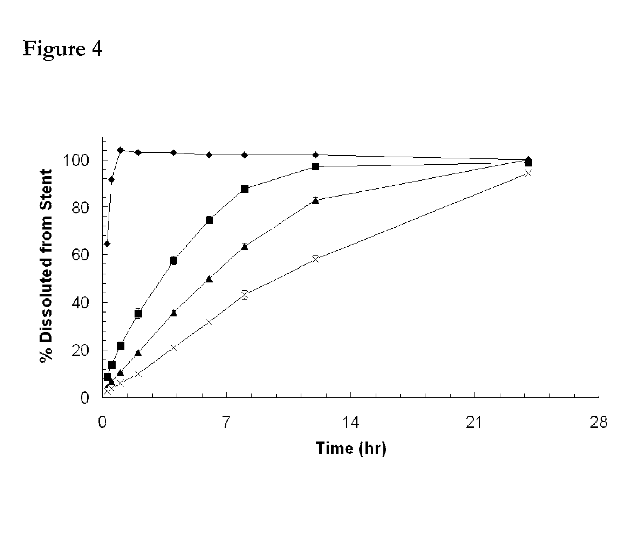

The kinetics of zotarolimus dissolution from stents into the surrounding medium is shown in **Figure 4****,** where diamonds indicate no topcoat, squares indicate 2 µg/mm topcoat; triangles indicate 5 µg/mm topcoat, and "X's" indicate 10 µg/mm topcoat; standard error of the means are also shown. The results showed that drug elution was fastest from stents without any PC topcoat (0 µg/mm), and could be increasingly slowed through application of topcoats of increasing thickness.

### Example 8 Neointimal formation in vivo after stent implantation

A porcine coronary overstretch model study (Schwartz, 1992) was conducted to examine neointimal formation for 28 days following stent implantation. The study evaluated a number of drug-eluting stents randomized *vs*. comparator stents containing only a polymer coating without drug (TriMaxx™).

### Experimental design and methods

In each pig, the three major coronary arteries were implanted with one test stent each. Additionally, three pigs were implanted with three non-drug containing TriMaxx™ stents (Abbott Laboratories; Abbott Park, IL) each (9 total stents) for comparison. The stents that were compared included ZoMaxx™ stents (3.0 x 15 mm), commercially available rapamycin (8.5 µg/mm or 1.40 µg/mm²)-polymer coated Cypher® stents (3.0 x 13 mm; Cordis Corp.; Miami, FL) and paclitaxel-(6.8 µg/mm or 1.0 µg/mm²) polymer coated Taxus® stents (3.0 x 16 mm; Boston Scientific; Natick, MA) stents.

Stents were implanted with a balloon/artery ratio of 1:1.30 as determined by quantitative coronary angiography. There were no cardiac- or stent- related mortalities in the study. After 28 days, animals were euthanized, and the hearts were removed and perfusion fixed at ~100 mm Hg with lactated Ringer's solution until cleared of blood, followed by 10% neutral buffered formalin. Stented vessels were excised, then infiltrated and embedded in methylmethacrylate (MMA). All blocks containing stented vessels were sectioned so that three, in-stent sections and two control sections (proximal and distal to the stent) were taken. Two serial thin sections (approximately 5 µm) were taken at each level and stained with hematoxylin and eosin (HE) and Masson's Verhoeff Elastin (MVE). Sections were evaluated and scored using the BIOQUANT TCW98 image analysis system (Bioquant; Nashville, TN).

### Results

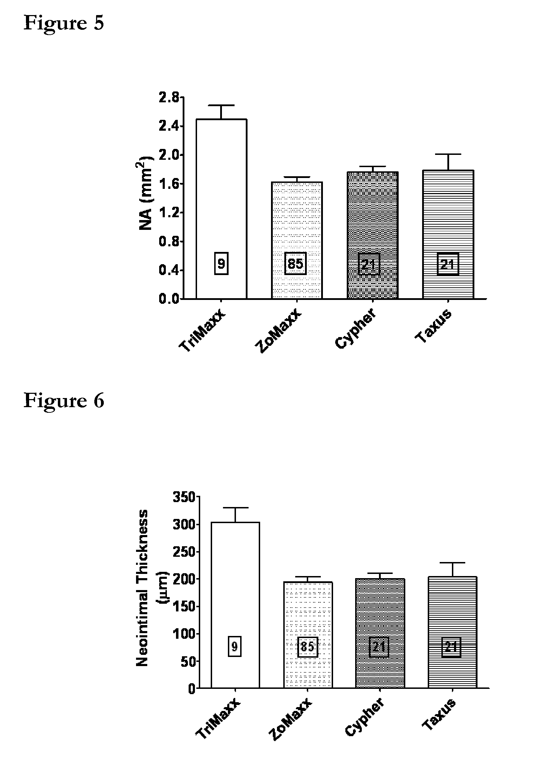

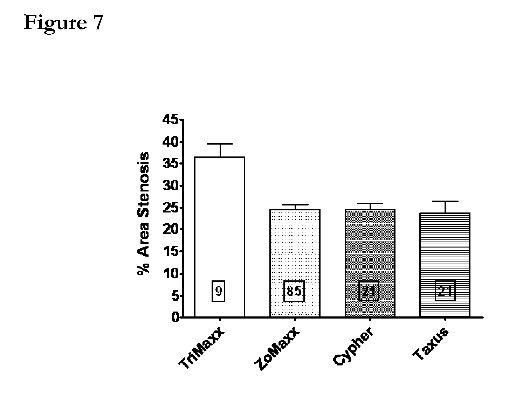

Average values for all stents within the three drug-eluting groups for neointimal area, neointimal thickness, and percent-area stenosis are shown in **Figures 5-7****,** respectively (represented as means ± s.e.m.; *p* values were calculated *versus* TriMaxx; boxed numbers indicate the number of stents/group). ZoMaxx™, Cypher®, and Taxus® stents had statistically equivalent reductions in formation of neointima as represented by morphometric measurements compared to TriMaxx™ stents.

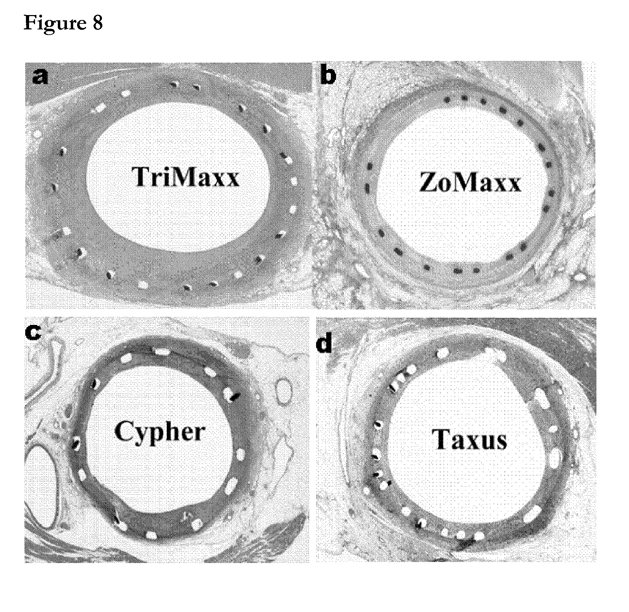

Each of the state-of-the-art, single drug stents, ZoMaxx™, Cypher®, and Taxus® showed dramatic reductions in neointimal formation *versus* TriMaxx™ controls. For example, the average reduction in neointima for ZoMaxx™ stents was 34.5% *versus* controls, as shown in Table 9. Pictorial representations are shown in Figures **8A-8C****,** which show micrographs that represent average neointimal areas for each group.

**TABLE 9**

| Improvements in morphometric measurements *vs*. TriMaxx™ non-drug eluting stents | | | | |
|---|---|---|---|---|
| Stent | Neointimal Area (mm²) | Neointimal Thickness (µm) | % Area Stenosis | Average |
| ZoMaxx™ | 34.7% | 36.0% | 32.7% | 34.5% |

### Example 9 Comparison of the elution profiles, tissue concentration and blood concentration of zotarolimus-polymer coated stents (ZoMaxx™) and rapamycin-coated stents (Cypher®) implanted in rabbit iliac arteries

The objective of these studies was to assess the distribution of rapamycin eluted from the Cypher® stent and compared to zotarolimus eluted from the ZoMaxx^{™} stents. Desired profiles for each stent-drug combinations is efficient elution from the support, local delivery to tissues situated adjacent to the stent, and low blood concentration to minimize systemic side effects.

Stents coated with approximately 10 µg/mm of either zotarolimus or rapamycin (ZoMaxx^{™} and Cypher® stents, respectively) were implanted into the iliac arteries of rabbits, and assays that determined percent elution from the stents **(****Figure 9****),** the amount of tissue penetration by the drugs **(****Figures 10** **and** **11** **(****Figure 10** compares these results with a similar study conducted in pigs)) and the whole blood concentration of the drugs **(****Figure 11****)** were assayed. The data derived for Figure 10 is reported in Table 10

**TABLE 10**

| Concentration in tissue, ng/g Cypher® data from (Carter *et al.,* 2004). | | | | |
|---|---|---|---|---|
| Time (Hours) | ZoMaxx™ Pig | Cypher® Pig | ZoMaxx™ Rabbit | Cypher® Rabbit |
| 6 | 42596 | | 71180 | 5950 |
| 12 | 39719.6 | | | |
| 24 | 113776.7 | 4920 | 30570 | 3660 |
| 36 | 64862.9 | | | |
| 48 | 68223.4 | | 62670 | 5970 |
| 72 | 110414.3 | | 115850 | 4470 |
| 120 | 39124.1 | | 18720 | 2570 |
| 168 | 25613 | | 47370 | 2380 |
| 192 | | 2220 | | |
| 336 | 8207.6 | 3520 | 48260 | 2190 |
| 672 | 8140.4 | | 10850 | 1310 |
| 720 | | 1200 | | |
| 1440 | | 930 | | |
| 2160 | | 890 | | |

### Drug elution study

At the indicated times points in **Figure 9****,** stents (n=4) were removed and the amount of drug left on the stent assayed as in Example 7. The data were then averaged, and plotted in the graph shown in **Figure 9****,** where filled circles represent the data points for ZoMaxx^{™} and the filled squares represent the data points for Cypher®. The error bars represent S.E.M. The *x*-axis represents time in days, while the *y*-axis represents percent of drug eluted.

As shown in **Figure 9****,** at early time points, a greater percentage of rapamycin was eluted from the stents than zotarolimus up to about 7 days, at which time the release of zotarolimus was accelerated compared to rapamycin. Rapamycin eluted faster and earlier than zotarolimus, although the overall trends for both drugs were similar.

### Drug penetration study

At the indicated times points in **Figure 10****,** stents (n=4) were removed and the amount of drug that had penetrated the arterial walls adjacent to the stent was assayed for the presence of the drugs. The data were then averaged, and plotted in the graph shown in **Figure 10****,** where filled circles represent the data points for ZoMaxx^{™} and the filled squares represent the data points for Cypher®. The error bars represent S.E.M. The *x-*axis represent time in days, and the *y*-axis represent the amount of drug in tissue expressed as µg/g tissue.

As dramatically shown in **Figure 10****,** at all times, the tissue concentration of zotarolimus eluted from the ZoMaxx^{™} stent was significantly greater than the tissue concentration of rapamycin eluted from the Cypher® stent. At all time points, the amount of rapamycin never exceeded 10 µg/g of tissue, while the amount of zotarolimus fluctuated from a high of slightly less than 120 µg/g of tissue at 5 days after implantation, to a low of slightly greater than 10 µg/g tissue at 28 days after implantation.

Similar results are obtained when repeated in pigs **(****Figure 11****;** here the *x*-axis is shown in hours, and the *y*-axis as log mean concentration (ng/g)); filled squares, ZoMaxx^{™}/pig; filled triangles, Cypher®/pig; upside-down filled triangle, ZoMaxx^{™}/rabbit; side-ways filled triangle, Cypher®/rabbit. Again, at all time points and despite the model system, the concentration of rapamycin eluted from the Cypher® stent was less than that of zotarolimus eluted from the ZoMaxx^{™} stent.

### Blood concentration study

At the indicated times points in **Figure 12****,** blood samples were collected by venipuncture into evacuated collection tubes containing edetic acid (EDTA) (n=4). Blood concentrations of zotarolimus and rapamycin were determined using a validated liquid/liquid extraction HPLC tandem mass spectrometric method (LC-MS/MS) (Ji *et al.,* 2004). The lower limit of quantification of zotarolimus was 0.20 ng/ml using 0.3 ml blood sample. The data were then averaged, and plotted in the graph shown in **Figure 12****,** where filled circles represent the data points for ZoMaxx^{™} and the filled squares represent the data points for Cypher®. The error bars represent S.E.M. Time is plotted on the *x*-axis, while blood concentration of the drugs is represented on the *y*-axis in ng/ml.

As shown in **Figure 12****,** the concentration of zotarolimus eluted from the ZoMaxx^{™} stents in the blood was lower than rapamycin eluted from the Cypher® stents up to 28 days, where at 28 days, the concentration was approximately similar. At the earliest time points, the blood concentration of rapamycin is dramatically elevated (*e.g.,* approximately 8, 6 and 4 ng/ml at the three earliest time points) compared to zotarolimus. As time progresses, the blood concentration of zotarolimus continues to decrease from a high of 4 ng/ml (initial time point) to a low of approximately 0.5-1 ng/ml (5 days and after implantation).

### Conclusions

While the elution profile trends were similar for zotarolimus eluting from ZoMaxx^{™} stents and rapamycin eluting from Cypher® stents **(****Figure 9****),** higher concentrations of zotarolimus resides in the target tissues after implantation and remained high for over 14 days **(****Figure 10****)** this phenomenon was observed whether the ZoMaxx^{™} stents were implanted in pigs or rabbits **(****Figure 11****).** Consistent with this observation was the blood concentration of zotarolimus, which was always lower than that of rapamycin up to at least 14 days **(****Figure 12****).** Overall, these results indicate that zotarolimus delivered from ZoMaxx^{™} stents remains proximal to the site of implantation, while rapamycin delivered from Cypher® stents has a more systemic distribution, with lower concentrations in the target tissues adjacent to the stent.

### Example 10 Extended studies of zotarolimus-coated and rapamycin-coated stents in vivo

While the purpose of stents is to keep a body lumen pendent, implantation in blood vessels often results in neointima formation, which occludes the lumen, and provokes an inflammatory response. This study set out to determine the neointima and inflammatory profiles of the ZoMaxx^{™} stent compared to the Cypher® stent over an extended period of time.

### Neointima study

### The methods of Carter et al. (2004) were followed, and are summarized here.

Immediately following euthanasia, the hearts were harvested, and the coronary arteries were perfusion-fixed with 10% buffered formalin at 100 mm Hg. The stented coronary artery segments were processed for plastic embedding, staining and morphometric analysis of six sections from the proximal through the distal margin of the stent. The specimens were embedded in methyl methacrylate and sections were obtained. Sections were then polished, mounted on a glass slide and stained with metachromatic stain. All histopathologic analysis was completed by a single independent investigator blinded to treatment group. Vessel morphometry and morphologic analysis of inflammation and smooth muscle content were completed using published methods (Kornowski *et al.,* 1998; Schwartz, 1992; Suzuki *et al.,* 2001).

Inflammation was graded as 0, none; 1, scattered inflammatory cells; 2, inflammatory cells encompassing 50% of a strut in at least 25% to 50% of the circumference of the artery; 3, inflammatory cells surrounding a strut in at least 25% to 50% of the circumference of the artery.

### Statistical analysis

The morphometric measurements from each of the 4-stent sections were summed and divided by 4 to generate the mean value for each parameter within the stent. For continuous variables, including morphometric parameters, the mean differences between treatment groups were tested with ANOVA. For morphologic parameters, scores were assigned to each of the four sections within the stented segment, the median value used as the score for the stent. The data were ranked within each cohort (3, 30, 90, or 180 days) and stratified. An ANOVA was performed on these ranks. Categorical data were compared with chi-square analysis. Data are expressed as mean F S.D. unless otherwise stated.

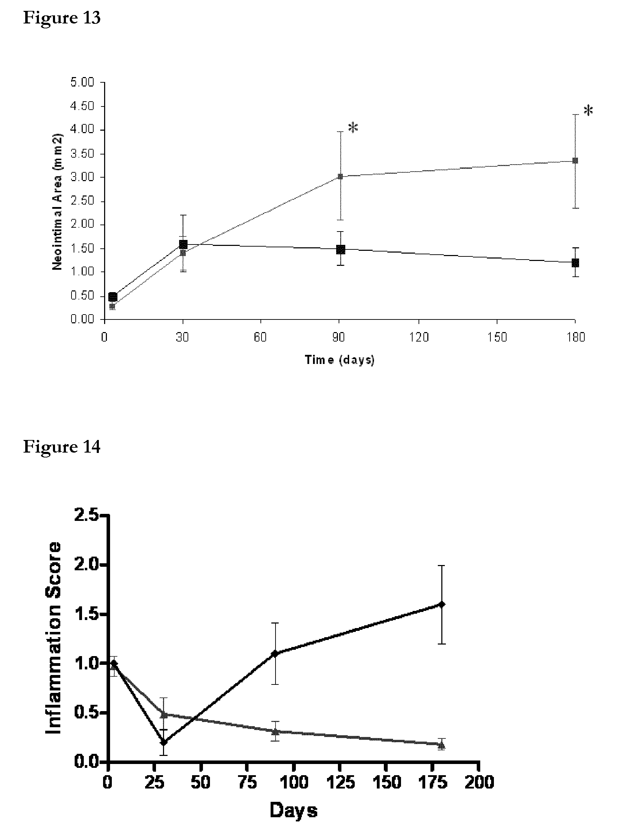

**Figure 13** shows the results of the neointimal area studies, wherein the *x*-axis represents time in days, and the *y*-axis represents the average neointimal area expressed in mm². The data obtained using the rapamycin coated Cypher® stent is shown by the small filled squares and is excerpted from (Carter *et al.,* 2004); data obtained from the zotarolimus coated ZoMaxx^{™} stent is shown by the large filled squares.

From 0-30 days after implantation, neointimal areas for both stents were similar (approximately 0.45 mm² for rapamycin and 0.50 mm² for zotarolimus 3 days after implantation, and approximately 1.45 mm² for rapamycin and 1.60 mm² for zotarolimus 30 days after implantation). However, at day 90, the differences between the two stents was startling: the neointimal area for those subjects receiving the zotarolimus-coated stents actually *decreased* to approximately 1.50 mm², while those receiving the rapamycin-coated stents *increased* dramatically to approximately 3.0 mm². This trend was echoed at 180 days after implantation, with rapamycin neointimal area approaching 3.25 mm² while the zotarolimus area decreased to approximately 0.85 mm².

**Figure 14** shows the results of the inflammation studies, wherein the *x*-axis represents time in days, and the *y*-axis represents the average inflammation score. The data obtained using the rapamycin coated Cypher® stent are shown by the filled diamonds and is excerpted from (Carter *et al.,* 2004); data obtained from the zotarolimus coated ZoMaxx^{™} stent is shown by the large filled triangles. The data are also shown in Table 11.

**TABLE 11**

| Inflammatory scores over time (n=10) | | | | |
|---|---|---|---|---|
| **ZoMaxx**™ | | | **Cypher®** | |
| Days | Mean | SD | Mean | SD |
| 3 | 0.970 | 0.315 | 1.000 | 0.000 |
| 30 | 0.490 | 0.510 | 0.200 | 0.420 |
| 90 | 0.315 | 0.315 | 1.100 | 0.990 |
| 180 | 0.180 | 0.190 | 1.600 | 1.260 |

Pigs implanted with ZoMaxx^{™} stents experienced a marked reduction in inflammation over time, with a steady, almost linear decrease in inflammation over time, from a high of 0.97 ± 0.315 at 3 days after implantation, to a low of 0.180 ± 0.190 at 180 days after implantation. In stark contrast, pigs receiving the Cypher® stent were less fortunate. While inflammation was quickly reduced at 30 days after implantation (0.200 ± 0.420) and was less than in those pigs with the ZoMaxx^{™} stent (0.490 ± 0.510), inflammation scores soared to a exceed those seen after initial transplantation, especially at 180 days (1.600 ± 1.260), exceeding those scores for the ZoMaxx^{™} by almost 10-fold (0.180 *v*. 1.6).

### Conclusion

Pigs receiving zotarolimus delivered from ZoMaxx^{™} stents show significantly less neointima formation and reduced inflammation over the long run than those receiving rapamycin delivered from Cypher® stents.

### Example 11 28 day overlapping drug-eluting stent study

This example set out to more thoroughly explore long-term efficacy of implanted stents as observed in Example 10. This example differs from Example 10 in that an additional stent was added for comparison (in this case, eluting paclitaxel) and the study was performed in rabbit iliac arteries. In addition to monitoring neointima formation, other parameters were measured, including morphometric analyses, fibrin deposition, granuloma and giant cell reactions, inflammation scores. The experimental set-up is shown in Table 12; in **Figure 15D****,** the placement of the stents is diagrammed.

**TABLE 12**

| Experimental design for long-term study comparing three different stent:drug combinations | | | | |
|---|---|---|---|---|
| | Light microscopy | | Ultrastructure (SEM) | |
| | Arteries | Animals | Arteries | Animals |
| Cypher® (28 days) | 10 | 5 | 6 | 3 |
| Taxus® (28 days) | 10 | 5 | 6 | 3 |
| ZoMaxx™ (28 days) | 10 | 5 | 6 | 3 |
| Cypher® (90 days) | 6 | 3 | n/a | n/a |
| Taxus® (90 days) | 6 | 3 | n/a | n/a |
| ZoMaxx™ (90 days) | 6 | 3 | n/a | n/a |

The results for after 28 days after implantation are shown in **Figures 15A-C.** When endothelialization of the implanted stents was measured, ZoMaxx^{™} stents were almost confluent with endothelial cells, and significantly more so (p<0.05) than either Cypher® or Taxus® stents **(****Figure 15A****).** Likewise, the percent of red blood cells, a measure of injury, was less than 15% with ZoMaxx^{™}, while Cypher® implanted stent areas approached 32% and Taxus® was over 40% and significantly different (p<0.01) than ZoMaxx^{™} **(****Figure 15B****).** Finally, fibrin deposition, another assay of injury, was significantly less for ZoMaxx^{™}-implanted stents (<25%) than for either Cypher® (40%; p<0.05) and Taxus® (approximately 65%; p<0.001) **(****Figure 15C****).**

### Example 12 Clinical example

ZoMaxx IVUS, a clinical, angiographic and intravascular ultrasound (IVUS) trial was conducted in 40 patients with a mean age of 59 years (+/- 9). Eighty percent of patients had hyperlipidemia, while 40 percent had diabetes, and 40 percent had a prior heart attack. The mean lesion length stented was 14.4 mm (+/- 3.3). Before treatment with a ZoMaxx stent, percent diameter stenosis (DS), the percent of vessel blocked with disease, was 70% (+/- 10). Directly after treatment with ZoMaxx, percent diameter stenosis improved to 5.1 percent (+/- 5.3) inside the stent (in-stent) and 19 percent (+/- 7) in-segment. Late lumen loss, a measure of the change in vessel diameter between the time immediately following stent placement and at four-months, was 0.20 mm (+/- 0.35) in-stent and 0.17 mm (+/- 0.35) in-segment. In-stent net volume obstruction, the amount of blockage that re-formed inside the stent in the four-months following the initial procedure, was 6.5 percent (+/- 6.2). The ZoMaxx stent was delivered with 100 percent success, and no major adverse cardiac events (MACE) occurred during the procedure or during the four months of follow-up.

### Example 13 (prophetic) Clinical application

The introduction and subsequent widespread use of stents that deliver single anti-proliferative agents has reduced the restenosis rate to less than 10% in the general clinical population. However, a clear rationale exists for the delivery of appropriate drug combinations from stents to treat patients both in the general clinical population and from a variety of cardiovascular disease subsets to reduce restenosis rates and adverse clinical events still further. For example, it is well accepted that the rate of restenosis is significantly increased in stented diabetic patients when compared to those without the disease, and that an inflammatory response to stenting exists in both diabetic and non-diabetic patients (Aggarwal *et al.,* 2003). In addition, inflammation is a hallmark in patients with acute coronary syndrome (ACS), a term which defines a range of acute myocardial ischemic conditions, including unstable angina, non-ST segment elevation myocardial infarction, as well as infarction associated with persistent ST-segment elevation. These patients are often prime candidates for stent deployment, and relative to the general patient population undergoing percutaneous intervention (PCI), have significantly higher rates of recurrent ischemia, reinfarction and subsequent need for repeat PCI procedures. Finally, obesity is often associated with a pro-inflammatory state and endothelial dysfunction. Both conditions are known to be independent predictors of early restenosis after coronary stent placement. In fact, a case has been made for an association between obesity, interleukin-6 (IL-6) production by adipocytes and coronary artery disease, suggesting a link between elevations of this inflammatory cytokine and the development of CAD in this sub-set of patients (Yudkin *et al.,* 2000).

Diabetic patients exhibit higher levels of the inflammatory marker, c-reactive protein (CRP) than non-diabetic patients (Aggarwal *et al.,* 2003; Dandona and Aljada, 2002). This protein has been clearly identified as a key inflammatory mediator in patients with coronary artery disease and is a predictor of adverse events in patients with severe unstable angina (Biondi-Zoccai *et al.,* 2003). CRP stimulates the production of monocyte chemo-attractant protein (MCP-1) by human endothelial cells. The release of this mediator is accompanied by the influx of monocytes, resulting in a marked inflammatory state as these cells are activated and move into the sub-endothelial space, where they form foam cells containing oxidized low-density lipoprotein (LDL). Plasma IL-6 and tumor necrosis factor-α (TNF-α) are inflammatory cytokines that are also elevated in the obese patient, and in type 2 diabetics. In fact, elevation of high-sensitivity CRP, IL-6 or serum vascular cell adhesion molecule-1 (VCAM-1) have been associated with increased mortality in patients with coronary artery diseases (Roffi and Topol, 2004). Since it has been shown that neointimal formation, a hallmark of the restenotic process, is accentuated by inflammation, the use of stents which deliver a combination of agents with anti-inflammatory and anti-proliferative activities including zotarolimus and paclitaxel to the local vessel environment would be expected to have clear utility in diabetic patients.

Disruption of an atheromatous plaque is central to the initiation of an acute coronary syndrome (Grech and Ramsdale, 2003). Plaque rupture can be induced by increased concentrations of matrix metalloproteinases secreted by foam cells, leading to plaque instability and ultimate rupture of the thin fibrous cap which overlies the developing lesion. In addition, tissue factor, which is expressed on the surface of foam cells, activates coagulation factor VII, which leads to the formation of thrombin. Generation of this protein leads to platelet activation and aggregation, as well as the conversion of fibrinogen to fibrin, and the clear formation of thrombus. Initial concern regarding the deployment of stents in this setting appears unfounded, since improvements in stent deployment and technique have shown that stented patients have less recurrent ischemia, reinfarction and need for repeat angioplasty (Grech and Ramsdale, 2003). The close relationship between inflammation and the development of coronary artery lesions makes the use of stents that deliver a combination of agents with anti-inflammatory and anti-proliferative activities including zotarolimus and paclitaxel to the local vessel environment an attractive approach to treating such patients.

The stents described herein will be deployed in patients who are diagnosed with ischemic heart disease due to stenotic lesions in coronary arteries and in subsets of the clinical population at higher risk for recurrent coronary disease and other adverse clinical events. Other targets for intervention include peripheral vascular diseases including stenoses in the superficial femoral arteries, renal arteries, iliacs, and vessels below the knee. Target vessels for interventional procedures will be reached using percutaneous vascular access via either the femoral or radial artery, and a guiding catheter will be inserted into the vessel. The target lesion will then be crossed with a guide wire, and the balloon catheter will be inserted either over the wire or using a rapid exchange system. The physician will determine the appropriate size of the stent to be implanted by online quantitative coronary angiography (QCA) or by visual estimate. The stent will be deployed using appropriate pressure as indicated by the compliance of the stent, and a post-procedure angiogram can then be obtained. When the procedure is completed, the patient will be regularly monitored for angina status and for the existence of any adverse events. The need for repeat procedures will also be assessed.

It is understood that the foregoing detailed description and accompanying examples are merely illustrative and are not to be taken as limitations upon the scope of the invention, which is defined solely by the appended claims and their equivalents. Various changes and modifications to the disclosed embodiments will be apparent to those skilled in the art. Such changes and modifications, including without limitation those relating to the chemical structures, substitutents, derivatives, intermediates, syntheses, formulations and/or methods of use of the invention, can be made without departing from the spirit and scope thereof.

### References

Aggarwal, A., D.J. Schneider, B.E. Sobel, and H.L. Dauennan. 2003. Comparison of inflammatory markers in patients with diabetes mellitus versus those without before and after coronary arterial stenting. Am J Cardiol. 92:924-9.
Baker, H., A. Sidorowicz, S.N. Sehgal, and C. Vezina. 1978. Rapamycin (AY-22,989), a new antifungal antibiotic. III. In vitro and in vivo evaluation. J Antibiot (Tokyo). 31:539-45.
Berg, E., R. Tuch, M. Dror, and R. Wolff. US Patent 5,464,650. 1995. Intravascular stent and method.
Bierer, B.E., S.L. Schreiber, and S.J. Burakoff. 1991. The effect of the immunosuppressant FK-506 on alternate pathways ofT cell activation. Eur J Immunol. 21:439-45.
Biondi-Zoccai, G.G., A. Abbate, G. Liuzzo, and L.M. Biasucci. 2003. Atherothrombosis, inflammation, and diabetes. J Am Coll Cardiol. 41:1071-7.
Bowers, R., S. Jones, and P. Stratford. US Patent 6,090,901. 2000. Polymeric surface coatings.
Bowers, R., S. Jones, P. Stratford, and S. Charles. US Patent 5,705,583. 1998. Polymeric surface coatings.
Brown, E.J., M.W. Albers, T.B. Shin, K. Ichikawa, C.T. Keith, W.S. Lane, and S.L. Schreiber. 1994. A mammalian protein targeted by G1-arresting rapamycin-receptor complex. Nature. 369:756-8.
Bunchman, T.E., and C.A. Brookshire. 1991. Smooth muscle cell proliferation by conditioned media from cyclosporine-treated endothelial cells: a role of endothelin. Transplant Proc. 23:967-8.
Carter, A.J., M. Aggarwal, G.A. Kopia, F. Tio, P.S. Tsao, R. Kolata, A.C. Yeung, G. Llanos, J. Dooley, and R. Falotico. 2004. Long-term effects of polymer-based, slow-release, sirolimus-eluting stents in a porcine coronary model. Cardiovasc Res. 63:617-24.
Caufield. US Patent No. 5,023,262. 1991. Hydrogenated Rapamycin Derivatives.
Caufield. WO 92/05179. 1992. Carboxylic Acid Esters of Rapamycin.
Dandona, P., and A. Aljada. 2002. A rational approach to pathogenesis and treatment of type 2 diabetes mellitus, insulin resistance, inflammation, and atherosclerosis. Am J Cardiol. 90:27G-33G.
Ding, N., and M. Helmus. US Patent 6,284,305. 2001. Drug coating with topcoat.
Ding, N., and M. Helmus. US Patent 6,358,556. 2002. Drug release stent coating.
Dumont, F.J., M.R. Melino, M.J. Staruch, S.L. Koprak, P.A. Fischer, and N.H. Sigal. 1990. The immunosuppressive macrolides FK-506 and rapamycin act as reciprocal antagonists in murine T cells. J Immunol. 144:1418-24.
Eng. US Patent No. 4,401,653. 1983. Combination of Rapamycin and Picibanil for the Treatment of Tumors.
Eury, R., D. Garguilo, and P. Villareal. US Patent No. 5,605,696. 1997. Drug loaded polymeric material and method of manufacture
Failli. EPO 467606. 1992a. Rapamycin Derivatives.
Failli. US Patent No. 5,120,842. 1992b. Silyl Ethers of Rapamycin.
Failli. US Patent No. 5,177,203. 1993. Rapamycin 42-Sulfonates and 42-(N-Carboalkoxy) Sulfamates Useful as Imunosuppressie Agents.
Fretz, H., M. Albers, A. Gala, R. Standaert, W. Lane, S. Burakoff, B. Bierer, and S. Schreiber. 1991. Rapamycin and FK506 binding proteins (immunophilins). J. Am. Chem. Soc. 113:1409-1411.
Froix, M. US Patent 5,163,852. 1992. Expandable polymeric stent with memory and delivery apparatus and method
Grech, E.D., and D.R. Ramsdale. 2003. Acute coronary syndrome: unstable angina and non-ST segment elevation myocardial infarction. Bmj. 326:1259-61.
Harding, M.W., A. Galat, D.E. Uehling, and S.L. Schreiber. 1989. A receptor for the immunosuppressant FK506 is a cis-trans peptidyl-prolyl isomerase. Nature. 341:758-60.
Hayward, C., D. Yohannes, and S. Danishefsky. 1993. Total synthesis of rapamycin via a novel titanium-mediated aldol macrocyclization reaction. J. Am. Chem. Soc. 115:9345-9346.
Helmus, M. 1990. Medical Device Design--A Systems Approach: Central Venous Catheters. In 22nd International Society for the Advancement of Material and Process Engineering Technical Conference.
Helmus, M., M. Tolkoff, and C. Raleigh. US Patent 5,447,724. 1995. Medical device polymer.
Higuchi, T., and V. Stella. 1987. Pro-drugs as Novel Delivery systems.
Igaki, K. US Patent No. 6,413,272. 2002. Stent for vessel.
Ji, Q., M. Reimer, and T. El-Shourbagy. 2004. 96-Well liquid-liquid extraction liquid chromatography-tandem mass spectrometry method for the quantitative determination of ABT-578 in human blood samples. Journal of Chromatography B. 805:67-75.
Kao. US Patent No. 5,120,725. 1992a. Bicyclic Rapamycins.
Kao. US Patent No. 5,120,727. 1992b. Rapamycin Dimers.
Kino, T., N. Inamura, F. Sakai, K. Nakahara, T. Goto, M. Okuhara, M. Kohsaka, H. Aoki, and T. Ochiai. 1987. Effect of FK-506 on human mixed lymphocyte reaction in vitro. Transplant Proc. 19:36-9.
Kopia, G., G. LLanos, and R. Falotico. WO0187372. 2001. Drug combinations useful for prevention of restenosis.
Kornowski, R., M.K. Hong, F.O. Tio, O. Bramwell, H. Wu, and M.B. Leon. 1998. In-stent restenosis: contributions of inflammatory responses and arterial injury to neointimal hyperplasia. J Am Coll Cardiol. 31:224-30.
Lewis, A., and S. Leppard. WO 02/055122. 2002. Sten[t]s with drug-containing amphiphilic polymer coating.
Lewis, A., and S. Leppard. US Patent Application Publication US 2005/004661. 2005. Sten[t]s with drug-containing amphilic polymer coating.
Luly. 1995. Macrocyclic Immunomodulators.
MacGregor, D. US Patent 4,994,071. 1991. Bifurcating stent apparatus and method.
Martel, R.R., J. Klicius, and S. Galet. 1977. Inhibition of the immune response byrapamycin, a new antifungal antibiotic. Can J Physiol Pharmacol. 55:48-51.
Mollison, K., A. LeCaptain, S. Burke, K. Cromack, P. Tarcha, Y.-C.J. Chen, and J. Toner. US Patent Application Publication 20030129215. 2003. Medical devices containing rapamycin analogs
Morris, R. 1992. Rapamycins: antifungal, antitumor, antiproliferative, and immunosuppressive macrolides. Transplant. Rev. 6:39-87.
Morris, R., and B. Meiser. 1989. Identification of a new pharmacologic action for an old compound. Med. Sci. Res. 17:609.
Nicolaou, K., T. Chakraborty, A. Piscopio, N. Minowa, and P. Bertinato. 1993. Total synthesis of rapamycin. J. Am. Chem. Soc. 115:4419-4420.
Okuhara, M., T. Hirokazu, G. Toshio, K. Tohru, and H. Hiroshi. EP Patent No. 0184162. 1986. Tricyclo compounds, a process for their production and a pharmaceutical composition containing the same.
Paiva, N.L., A.L. Demain, and M.F. Roberts. 1991. Incorporation of acetate, propionate, and methionine into rapamycin by Streptomyces hygroscopicus. J Nat Prod. 54:167-77.
Pinchuk, L. US Patent 5,092,877. 1992. Radially expandable endoprosthesis.
Rakhit. US Patent No. 4,316,885. 1982. Acyl Derivatives of Rapamycin.
Roche, E. 1987. Bioreversible Carriers in Drug Design. American Pharmaceutical Association and Pergamon Press.
Roffi, M., and E.J. Topol. 2004. Percutaneous coronary intervention in diabetic patients with non-ST-segment elevation acute coronary syndromes. Eur Heart J. 25:190-8.
Romo, D., S. Meyer, D. Johsnon, and S. Schrieber. 1993. Total synthesis of (-)-rapamycin using an Evans-Tishchenko fragment coupling. J. Am. Chem. Soc. 115:7906-7907.
Sabatini, D.M., H. Erdjument-Bromage, M. Lui, P. Tempst, and S.H. Snyder. 1994. RAFT1: a mammalian protein that binds to FKBP12 in a rapamycin-dependent fashion and is homologous to yeast TORs. Cell. 78:35-43.
Sahatjian, R. US Patent 5,304,121. 1994. Drug delivery system making use of a hydrogel polymer coating.
Schwartz, R. 1992. Restenosis and the proportional neointimal response to coronary artery injury: results in a porcine model. J Am Coll Cardiol. 19:267-274.
Sehgal, S.N. US Patent No. 3,929,992. 1975. Rapamycin and Process of Preparation.
Sehgal, S.N. US Patent No. 3,993,749. 1976. Rapamycin and Process of Preparation.
Sehgal, S.N., H. Baker, C.P. Eng, K. Singh, and C. Vezina. 1983. Demethoxyrapamycin (AY-24,668), a new antifungal antibiotic. J Antibiot (Tokyo). 36:351-4.
Sehgal, S.N., H. Baker, and C. Vezina. 1975. Rapamycin (AY-22,989), a new antifungal antibiotic. II. Fermentation, isolation and characterization. J Antibiot (Tokyo). 28:727-32.
Shichiri, M., Y. Hirata, T. Nakajima, K. Ando, T. Imai, M. Yanagisawa, T. Masaki, and F. Marumo. 1991. Endothelin-1 is an autocrine/paracrine growth factor for human cancer cell lines. J Clin Invest. 87:1867-71.
Siekierka, J.J., S.H. Hung, M. Poe, C.S. Lin, and N.H. Sigal. 1989. A cytosolic binding protein for the immunosuppressant FK506 has peptidyl-prolyl isomerase activity but is distinct from cyclophilin. Nature. 341:755-7.
Stack, R., H. Clark, W. Wlaker, and J. McElhaney. US Patent No. 5,527,337. 1996. Bioabsorbable stent and method of making the same.
Stella. US Patent No. 4,650,803. 1987. Prodrugs of Rapamycin.
Surendra. US Patent No. 4,885,171. 1989. Use of Rapamycin in Treatment of Certain Tumors.
Suzuki, T., G. Kopia, S. Hayashi, L.R. Bailey, G. Llanos, R. Wilensky, B.D. Klugherz, G. Papandreou, P. Narayan, M.B. Leon, A.C. Yeung, F. Tio, P.S. Tsao, R. Falotico, and A.J. Carter. 2001. Stent-based delivery of sirolimus reduces neointimal formation in a porcine coronary model. Circulation. 104:1188-93.
Tang, R., F. Mares, W. Boyle Jr., T.-H. Chiu, and K. Patel. US Patent 4,916,193. 1990. Medical devices fabricated totally or in part from copolymers of recurring units derived from cyclic carbonates and lactides
Tuch, R. US Patent 5,624,411. 1997. Intravascular stent and method.
Vezina, C., A. Kudelski, and S.N. Sehgal. 1975. Rapamycin (AY-22,989), a new antifungal antibiotic. I. Taxonomy of the producing streptomycete and isolation of the active principle. J Antibiot (Tokyo). 28:721-6.
Yamagishi, S., C.C. Hsu, K. Kobayashi, and H. Yamamoto. 1993. Endothelin 1 mediates endothelial cell-dependent proliferation of vascular pericytes. Biochem Biophys Res Commun. 191:840-6.
Yang, D., J. Stanslaski, and L. Wang. US Patent 6,419,692. 2002. Surface protection method for stents and balloon catheters for drug delivery
Yudkin, J.S., M. Kumari, S.E. Humphries, and V. Mohamed-Ali. 2000. Inflammation, obesity, stress and coronary heart disease: is interleukin-6 the link? Atherosclerosis. 148:209-14.

For reasons of completeness, various aspects of the invention are set out in the following numbered clauses:
Clause 1. A drug delivery system, comprising
   a supporting structure comprising a pharmaceutically acceptable carrier or excipient; and
   a first therapeutic composition comprising zotarolimus or prodrugs, derivatives, esters, salts thereof,
   wherein, when the system is implanted in a body lumen of a subject, delivery of zotarolimus to a lumen wall adjacent to the system is greater than that when compared to delivery of a control therapeutic composition from a control drug delivery system containing a similar dose to the first therapeutic composition. Clause 2. The system of clause 1, wherein the control therapeutic composition comprises an olimus drug.
Clause 3. The system of clause 2, wherein the olimus drug comprises one selected from the group consisting of everolimus, rapamycin, tacrolimus (FK506), biolimus A9, CCI-779, RAD 001, AP23573 and combinations thereof.
Clause 4. The system of clause 1, wherein the control therapeutic composition comprises an anti-inflammatory.
Clause 5. The system of clause 4, wherein the anti-inflammatory comprises one selected from the group consisting of dexamethasone hydrocortisone, estradiol, acetaminophen, ibuprofen, naproxen, fluticasone, clobetasol, adalimumab, sulindac, and combinations thereof.
Clause 6. The system of clause 1, wherein zotarolimus delivery to the lumen wall is increased for at least 28 days after implantation when compared to the control. Clause 7. The system of clause 1, wherein a cumulative percentage of zotarolimus eluted from the system is significantly greater than a cumulative percentage of rapamycin eluted from the control drug delivery system containing rapamycin 28 days after implantation.
Clause 8. The system of clause 1, wherein delivery of zotarolimus to the lumen wall results in a tissue concentration at least 5-fold greater than that of the control therapeutic 14 days or less after implantation of the systems.
Clause 9. The system of clause 8, wherein delivery of zotarolimus to the lumen wall results in a tissue concentration at least 10-fold greater than that of the control therapeutic.
Clause 10. The system of clause 1, wherein the body lumen is a blood vessel lumen, and implantation of the system comprising zotarolimus correlates with a reduction of neointima hyperplasia when compared to the control drug delivery system containing the second therapeutic composition at greater than or equal to three months after implantation.
Clause 11. The system of clause 10, wherein neointima hyperplasia is reduced by ≥ 60% when compared to the control drug delivery system 180 days after implantation.
Clause 12. The system of clause 10, wherein neointima hyperplasia is reduced by ≥ 30% when compared to the control drug delivery system 90 days after implantation.
Clause 13. The system of clause 1, wherein inflammation is significantly reduced when compared to the control drug delivery system containing a second therapeutic by at least 56 days after implantation.
Clause 14. The system of clause 13, wherein inflammation is significantly reduced by at least 182 days after implantation.
Clause 15. The system of clause 1, wherein endothelialization is significantly favored in a stent overlap study when compared to control drug delivery system containing rapamycin 28 days after implantation.
Clause 16. The system of clause 1, wherein fibrin production is significantly reduced when compared to control drug delivery system containing rapamycin 28 days after implantation in a stent overlap study.
Clause 17. The system of clause 1, wherein the drug delivery system comprises a stent, and the control drug delivery system comprises a stent.
Clause 18. The system of clause 17, wherein the concentration of zotarolimus is 10 µg/mm of the stent, and the concentration of the control therapeutic composition is 10 µg/mm of the stent.
Clause 19. The system of clause 18, wherein the control therapeutic composition comprises rapamycin.
Clause 20. The system of clause 1, further comprising a second therapeutic composition.
Clause 21. The system of clause 20, wherein the second therapeutic composition comprises at least one selected from the group consisting of anti-proliferative agents, anti-platelet agents, anti-inflammatory agents, anti-thrombolytic and anti-thrombotic agents.
Clause 22. The system of clause 21, wherein the anti-inflammatory agent is one selected from the group consisting of dexamethasone hydrocortisone, estradiol, acetaminophen, ibuprofen, naproxen, fluticasone, clobetasol, adalimumab and sulindac.
Clause 23. The system of clause 21, wherein the second therapeutic substance comprises an antibody.
Clause 24. The system of clause 1, wherein the subject is a pig or a rabbit.
Clause 25. The system of clause 1, wherein the subject is a human.
Clause 26. A drug delivery system, comprising
   a supporting structure comprising a pharmaceutically acceptable carrier or excipient; and
   a first therapeutic composition comprising zotarolimus or prodrugs, derivatives, esters, salts thereof,
   wherein, when the system is implanted in a body lumen of a subject, neointima hyperplasia is significantly reduced when compared to delivery of a control therapeutic composition from a control drug delivery system containing a similar dose to the first therapeutic composition at 90 days or greater after implantation.
Clause 27. The system of clause 26, wherein the control therapeutic composition comprises an olimus drug.
Clause 28. The system of clause 27, wherein the olimus drug comprises one selected from the group consisting of everolimus, rapamycin, tacrolimus (FK506), biolimus A9, CCI-779, RAD 001, AP23573 and combinations thereof.
Clause 29. The system of clause 26, wherein the control therapeutic composition comprises an anti-inflammatory.
Clause 30. The system of clause 29, wherein the anti-inflammatory comprises one selected from the group consisting of dexamethasone hydrocortisone, estradiol, acetaminophen, ibuprofen, naproxen, fluticasone, clobetasol, adalimumab, sulindac, and combinations thereof.
Clause 31. The system of clause 26, wherein zotarolimus delivery to the lumen wall is increased for at least 28 days after implantation when compared to the control.
Clause 32. The system of clause 26, wherein a cumulative percentage of zotarolimus eluted from the system is significantly greater than a cumulative percentage of rapamycin eluted from the control drug delivery system containing rapamycin 28 days after implantation.
Clause 33. The system of clause 26, wherein delivery of zotarolimus to a lumen wall adjacent to the system is greater than that when compared to delivery of a control therapeutic composition from a control drug delivery system containing a similar dose to the first therapeutic composition.
Clause 34. The system of clause 26, wherein delivery of zotarolimus to the lumen wall results in a tissue concentration at least 5-fold greater than that of the control therapeutic 14 days or less after implantation of the systems.
Clause 35. The system of clause 34, wherein delivery of zotarolimus to the lumen wall results in a tissue concentration at least 10-fold greater than that of the control therapeutic.
Clause 36. The system of clause 26, wherein neointima hyperplasia is reduced by ≥ 60% when compared to the control drug delivery system 180 days after implantation.
Clause 37. The system of clause 26, wherein neointima hyperplasia is reduced by ≥ 30% when compared to the control drug delivery system 90 days after implantation. Clause 38. The system of clause 26, wherein inflammation is significantly reduced when compared to the control drug delivery system containing a second therapeutic by at least 56 days after implantation.
Clause 39. The system of clause 38, wherein inflammation is significantly reduced by at least 182 days after implantation.
Clause 40. The system of clause 26, wherein the drug delivery system comprises a stent, and the control drug delivery system comprises a stent.
Clause 41. The system of clause 40, wherein the concentration of zotarolimus is 10 µg/mm of the stent, and the concentration of the control therapeutic composition is 10 µg/mm of the stent.
Clause 42. The system of clause 41, wherein the control therapeutic composition comprises rapamycin.
Clause 43. The system of clause 26, further comprising a second therapeutic composition.
Clause 44. The system of clause 43, wherein the second therapeutic composition comprises at least one selected from the group consisting of anti-proliferative agents, anti-platelet agents, anti-inflammatory agents, anti-thrombolytic and anti-thrombotic agents.
Clause 45. The system of clause 44, wherein the anti-inflammatory agent is one selected from the group consisting of dexamethasone hydrocortisone, estradiol, acetaminophen, ibuprofen, naproxen, fluticasone, clobetasol, adalimumab and sulindac.
Clause 46. The system of clause 43, wherein the second therapeutic substance comprises an antibody.
Clause 47. The system of clause 26, wherein the subject is a pig or a rabbit.
Clause 48. The system of clause 26, wherein the subject is a human.
Clause 49. A drug delivery system, comprising
   a supporting structure comprising a pharmaceutically acceptable carrier or excipient; and
   a therapeutic composition comprising zotarolimus or prodrugs, derivatives, esters, or salts thereof,
   wherein, when the system is implanted in a body lumen of a subject, inflammation is significantly reduced when compared to delivery of a control therapeutic composition from a control drug delivery system containing a similar dose to the first therapeutic composition at 90 days after implantation.
Clause 50. The system of clause 49, wherein the subject is a pig or rabbit.
Clause 51. The system of clause 49, wherein the subject is a human.
Clause 52. The system of clause 49, wherein the control therapeutic composition comprises an olimus drug.
Clause 53. The system of clause 52, wherein the olimus drug comprises one selected from the group consisting of everolimus, rapamycin, tacrolimus (FK506), biolimus A9, FK506, CCI-779, RAD 001, AP23573, and combinations thereof.
Clause 54. The system of clause 49, wherein the control therapeutic composition comprises an anti-inflammatory.
Clause 55. The system of clause 54, wherein the anti-inflammatory comprises one selected from the group consisting of dexamethasone hydrocortisone, estradiol, acetaminophen, ibuprofen, naproxen, fluticasone, clobetasol, adalimumab, sulindac, and combinations thereof.
Clause 56. The system of clause 49, wherein zotarolimus delivery to the lumen wall is increased for at least 28 days after implantation.
Clause 57. The system of clause 49, wherein a cumulative percentage of zotarolimus eluted from the system is significantly greater than a cumulative percentage of the control therapeutic composition eluted from the control drug delivery system 28 days after implantation.
Clause 58. The system of clause 49, wherein delivery of zotarolimus to the lumen wall results in a tissue concentration at least 5-fold greater than that of the control therapeutic 14 days or less after implantation of the systems.
Clause 59. The system of clause 58, wherein delivery of zotarolimus to the lumen wall results in a tissue concentration at least 10-fold greater than that of the control therapeutic.
Clause 60. The system of clause 49, wherein the body lumen is a blood vessel lumen, and implantation of the system comprising zotarolimus correlates with a reduction of neointima hyperplasia when compared to the control drug delivery system containing the second therapeutic composition at greater than or equal to three months after implantation.
Clause 61. The system of clause 60, wherein neointima hyperplasia is reduced by ≥ 60% when compared to the control drug delivery system 180 days after implantation.
Clause 62. The system of clause 60, wherein neointima hyperplasia is reduced by ≥ 30% when compared to the control drug delivery system 90 days after implantation.
Clause 63. The system of clause 49, wherein inflammation is significantly reduced by at least 56 days after implantation.
Clause 64. The system of clause 63, wherein inflammation is significantly reduced by at least 182 days after implantation.
Clause 65. The system of clause 49, wherein fibrin production is significantly reduced when compared to control drug delivery system containing rapamycin 28 days after implantation in a stent overlap study.
Clause 66. The system of clause 49, wherein the drug delivery system comprises a stent, and the control drug delivery system comprises a stent.
Clause 67. The system of clause 66, wherein the concentration of zotarolimus is 10 µg/mm of the stent, and the concentration of the control therapeutic composition is 10 µg/mm of the stent.
Clause 68. The system of clause 67, wherein the control therapeutic composition comprises rapamycin.
Clause 69. The system of clause 49, further comprising a second therapeutic composition.
Clause 70. The system of clause 69, wherein the second therapeutic composition comprises at least one selected from the group consisting of anti-proliferative agents, anti-platelet agents, anti-inflammatory agents, anti-thrombolytic and anti-thrombotic agents.
Clause 71. The system of clause 69, wherein the anti-inflammatory agent is one selected from the group consisting of dexamethasone hydrocortisone, estradiol, acetaminophen, ibuprofen, naproxen, fluticasone, clobetasol, adalimumab and sulindac.
Clause 72. The system of clause 69, wherein the second therapeutic substance comprises an antibody.
Clause 73. A method of treating a subject, comprising placing the system of clauses 1, 26 or 49 in a body lumen.
Clause 74. The method of clause 73, wherein the body lumen is a blood vessel lumen
Clause 75. The method of clause 49, wherein the subject is one selected from the group consisting of pig, rabbit or human.
Clause 76. A kit, comprising the system of clause 1, 26 or 49.
Clause 77. A drug delivery system, comprising
   a supporting structure capable of comprising a pharmaceutically acceptable carrier or excipient; and
   a therapeutic composition comprising zotarolimus or prodrugs, derivatives, esters, or salts thereof,
   wherein zotarolimus is significantly eluted from the supporting structure 30 days after implantation in a lumen of a blood vessel of a subject.
Clause 78. The system of clause 77, wherein the eluted zotarolimus comprises 85% to 100% of the zotarolimus loaded onto the device 15 - 30 days after implanting the device.
Clause 79. The system of clause 77, wherein the eluted zotarolimus is 5- to 15-fold more concentrated in walls of the blood vessel adjacent to the delivery system when compared to a control drug delivery system containing rapamycin.
Clause 80. The system of clause 77, wherein the control therapeutic composition is rapamycin, and the amount of zotarolimus in tissue is greater than rapamycin at the same time point.
Clause 81. The system of clause 77, wherein the control therapeutic composition is rapamycin, and the concentration of zotarolimus in blood is less than the concentration of rapamycin at the same time point.
Clause 82. The system of clause 77, wherein a concentration cₑ of eluted zotarolimus per unit of blood vessel wall adjacent to the delivery system is at time t in hours after implantation comprises:
   when 0 ≤ t < 120, then 6 µg/g ≤ cₑ ≤ 113 µg/g;
   when 120 ≤ t < 168, then 5 µg/g ≤ cₑ ≤ 40 µg/g;
   when 168 ≤ t < 720, then 2.5 µg/g ≤ cₑ ≤ 50 µg/g; and
Clause 83. The system of clause 77, wherein a whole blood concentration, c_{b}, of zotarolimus per ml of blood is at day d after implantation in a rabbit comprises:
   when 0 ≤ d ≤ 2, then 1.5 ≤ c_{b} ≤ 4;
   when 2 < d ≤ 3, then 1.4 ≤ c_{b} ≤ 1.5;
   when 3 < d ≤ 4, then 1.3 ≤ c_{b} ≤ 1.4; and
   when 4 < d ≤ 28, then 0 ≤ c_{b} ≤ 1.3
Clause 84. The system of clause 77, wherein a neointimal area of the blood vessel lumen implanted with the system is significantly less than the neotinimal area of the blood vessel lumen implanted with the control system at greater than or equal to 90 days.
Clause 85. The system of clause 84, wherein the neointimal area of the blood vessel lumen implanted with the system is less than or equal to 1.5 mm² 30 days or more after implantation in an over-stretch study.
Clause 86. The system of clause 77, wherein inflammation is significantly reduced 90 days or more after implantation of the system when compared to the control system. Clause 87. The system of clause 77, wherein endothelial cells covering a surface of the systems are significantly confluent 28 days after implantation of the system in an overlap rabbit model.
Clause 88. The system of clause 87, wherein significantly confluent comprises greater than 75% endothelialization.

## Claims

1. A stent comprising a coating, the coating comprising:
zotarolimus; and
a polymer,
wherein 85% to 100% of the total amount of zotarolimus present in the coating elutes 15-30 days after implantation of the stent.

2. Stent of claim 1 for use to deliver zotarolimus to a blood vessel, wherein a concentration, cₑ, of the zotarolimus per unit of blood vessel wall adjacent to the stent at time t in hours after implantation is:
when 0 < t < 120, then 6 µg/g < c_{c} < 113 µg/g;
when 120 < t < 168, then 5 µg/g < cₑ < 40 µg/g; and
when 168 < t < 720, then 2.5 µg/g < cₑ < 50 µg/g.

3. Stent of claim 1 for use for reducing neointima hyperplasia following implanting the stent in a blood vessel, wherein the neointima hyperplasia is reduced by ≥ 60% at 180 days after implantation when compared to a control drug delivery system containing rapamycin; or wherein the neointima hyperplasia is reduced by ≥ 30% at 90 days after implantation when compared to the control drug delivery system containing rapamycin.
